# EUROPÄISCHE PATENTSCHRIFT

(11) **EP 1 882 025 B1**
(45) Veröffentlichungstag und Bekanntmachung des Hinweises auf die Patenterteilung: **16.02.2011**
(21) Anmeldenummer: 06755135.8
(22) Anmeldetag: 10.05.2006
(51) Int. Cl.: C09K 19/32

(54) **2,6-NAPHTHYLRESTE ENTHALTENDE VERBINDUNGEN**
COMPOUNDS COMPRISING 2,6-NAPHTHYL GROUPS
COMPOSES CONTENANT DES GROUPES 2,6-NAPHTHYLE

(30) Priorität: 11.05.2005 DE 102005022642
(43) Veröffentlichungstag der Anmeldung: 30.01.2008
(73) Patentinhaber: BASF SE, 67056 Ludwigshafen (DE)
(72) Erfinder: ENGER, Olivier, 67067 Ludwigshafen (DE); SENS, Rüdiger, 67069 Ludwigshafen (DE); LENNARTZ, Christian, 67105 Schifferstadt (DE); PARKER, Robert, 68165 Mannheim (DE)
(86) Internationale Anmeldenummer: PCT/EP2006/062220
(87) Internationale Veröffentlichungsnummer: WO 2006/120220

(56) Entgegenhaltungen:
- EP-A- 0 332 409
- EP-A- 0 341 922
- EP-A- 0 982 621
- WO-A-99/19267
- DE-A1- 4 240 041

## Beschreibung

Die vorliegende Erfindung betrifft Verbindungen der allgemeinen Formel I 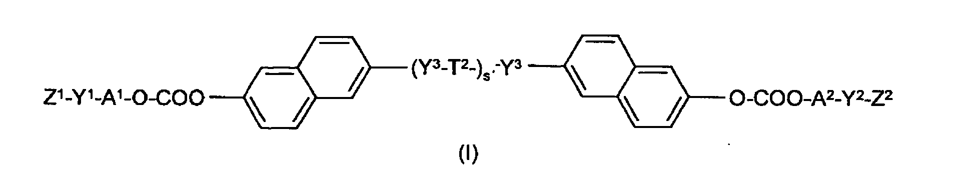 in welcher die Variablen folgende Bedeutung haben:
- Z¹, Z²: unabhängig voneinander Wasserstoff, gegebenenfalls substituiertes C₁-C₂₀-Alkyl, in welchem die Kohlenstoffkette durch Sauerstäffatome in Etherfunktion, Schwefelatome in Thioetherfunktion oder durch nichtbenachbarte Imino- oder C₁-C₄-Alkyliminogruppen unterbrochen sein kann, oder reaktive Reste, über die eine Polymerisation herbeigeführt werden kann,
- A¹, A²: unabhängig voneinander Spacer mit 1 bis 30 Kohlenstoffatomen, in welchen die Kohlenstoffkette durch Sauerstoffatome in Etherfunktion, Schwefelatome in Thioetherfunktion oder durch nichtbenachbarte Imino- oder C₁-C₄-Alkyliminogruppen unterbrochen sein kann,
- Y¹, Y²: unabhängig voneinander eine chemische Einfachbindung, Sauerstoff, Schwefel, -CO-, -O-CO-, -CO-O-, -S-CO-, -CO-S-, -NR-CO- oder -CO-NR-,
- Y³: eine chemische Einfachbindung, Sauerstoff, Schwefel, -CR=CR-, -C≡C-, -CR=CR-CO-O-, -O-CO-CR=CR-, -C≡C-O-, -O-C≡C-, -CH₂-CH₂-, -CH₂-O-, -O-CH₂-, -CH₂-S-, -S-CH₂-, -CO-, -O-CO-, -CO-O-, -S-CO-, -CO-S-, -NR-CO-, -CO-NR-, -O-CO-O-, -O-CO-NR- oder -NR-CO-O-,
- R: Wasserstoff oder C₁-C₄-Alkyl ,
- T²: zweiwertige gesättigte oder ungesättigte, gegebenenfalls substituierte iso- oder heterocyclische Reste,
und
- s': Werte von 0, 1 oder 2,
wobei für s' > 0 die Variablen Y³ und für s' > 1 die Variablen T² untereinander gleich oder voneinander verschieden sein können
und
sowohl die Wasserstoffatome im 2,6-Naphthylrest als auch die an Kohlenstoffatome gebundenen Wasserstoffatome in den Variablen Z¹, Z², A¹, A², Y³, R und T² teilweise oder vollständig durch Halogenatome substituiert sein können.

Weiterhin betrifft die Erfindung eine polymerisierbare oder nicht polymerisierbare flüssigkristalline Zusammensetzung, enthaltend eine oder mehrere der erfindungsgemäßen Verbindungen, ein Oligomer oder Polymer, erhältlich durch Oligomerisation oder Polymerisation einer polymerisierbaren, erfindungsgemäßen, flüssigkristallinen Zusammensetzung, ein Verfahren zum Bedrucken oder Beschichten eines Substrats durch Aufbringen einer polymerisierbaren, erfindungsgemäßen, flüssigkristallinen Zusammensetzung auf das Substrat und anschließende Polymerisation, die Verwendung der erfindungsgemäßen flüssigkristallinen Zusammensetzung oder des erfindungsgemäßen Oligomers oder Polymers zur Herstellung optischer oder elektrooptischer Bauteile, ein Verfahren zur Herstellung ausgewählter erfindungsgemäßer Verbindungen, sowie Zwischenprodukte, welche besonders zur Herstellung der ausgewählten erfindungsgemäßen Verbindungen geeignet sind.

Zahlreiche Verbindungen gehen beim Erwärmen vom kristallinen Zustand mit definierter Nah- und Femordnung der Moleküle nicht direkt in den flüssigen, ungeordneten Zustand über, sondern durchlaufen dabei eine flüssigkristalline Phase, in welcher die Moleküle zwar beweglich sind, die Molekülachsen jedoch eine geordnete Struktur ausbilden. Gestreckte Moleküle bilden hierbei oft nematische flüssigkristalline Phasen, die durch eine Orientierungsfemordnung durch Parallellagerung der Moleküllängsachsen gekennzeichnet sind. Enthält eine derartige nematische Phase chirale Verbindungen oder chirale Molekülteile, kann sich eine chiral-nematische bzw. cholesterische Phase ausbilden, welche durch eine helixartige Überstruktur gekennzeichnet ist.

Wegen ihrer bemerkenswerten optischen Eigenschaften sind flüssigkristalline Materialien, insbesondere nematische, chiral-nematische oder cholesterische Materialien, u.a. in optischen oder elektrooptischen Anwendungen von Interesse. Oft liegt jedoch der Temperaturbereich, in dem die flüssigkristalline Phase auftritt, außerhalb der gewünschten Anwendungstemperatur oder er erstreckt sich nur über ein kleines Temperaturintervall.

Will man die flüssigkristallinen Ordnungsstrukturen im festen Zustand fixieren, gibt es verschiedene Möglichkeiten. Neben glasartigem Erstarren beim Abkühlen aus dem flüssigkristallinen Zustand besteht die Möglichkeit des Einpolymerisierens in polymere Netzwerke oder, für den Fall, dass die flüssigkristallinen Verbindungen polymerisierbare Gruppen enthalten, der Polymerisation der flüssigkristallinen Verbindungen selbst.

Desweiteren ist oftmals eine möglichst hohe Doppelbrechung der flüssigkristallinen Materialien wünschenswert. Unter diesem Aspekt scheinen vor allem flüssigkristalline Materialien, welche 2,6-Naphthylreste enthalten, ein hohes Potenzial zu besitzen.

Polymerisierbare cholesterische Verbindungen, welche 2,6-Naphthylreste und reaktive Glycidylreste enthalten, sind beispielsweise in der Publikation von M.A. Espinosa et al., Journal of Polymer Science Part A, Polymer Chemistry, Vol. 39, 2847 - 2858 (2001), thermisch härtbare, nematische und cholesterische Diester, welche 2,6-Naphthylreste und reaktive Propargyl-Endgruppen aufweisen, in der Publikation von H.R. Kricheldorf und A. Gerken, High Performance Polymers 9 (1997), 75 - 90 sowie in der Schrift DE 197 17 371 A1 beschrieben. 2,6-Naphthylreste enthaltende, nicht polymerisierbare Verbindungen werden in der Schrift EP 0 982 621 A2, EP 0 332 409 A2, EP 0 341 922 A2 und DE 42 400 41 A1 offenbart.

Aufgabe der vorliegenden Erfindung war es, weitere für die Herstellung von flüssigkristallinen Zusammensetzungen geeignete Verbindungen mit vergleichsweise hohen doppelbrechenden Eigenschaften bereitzustellen, welche selbst oder in Form solcher Zusammensetzungen eine für die Verarbeitung ausreichend große Phasenbreite aufweisen.

Demgemäß wurden die eingangs beschriebenen Verbindungen der Formel I gefunden.

Für die Variablen Z¹ und Z² in Formel I kommen in Frage als C₁-C₂₀-Alkyl, dessen C₁-C₂₀-Kohlenstoffkette durch Sauerstoffatome in Etherfunktion unterbrochen sein kann, z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methyl-pentyl, Heptyl, Hept-3-yl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeich-nungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octa-decyl, Nonadecyl, Eicosyl, Methoxymethyl, 2-Ethylhexoxymethyl, 2-Methoxyethyl, 2-Ethoxyethyl, 2-Propoxyethyl, 2-Isopropoxyethyl, 2-Butoxyethyl, 2- oder 3-Methoxy-propyl, 2- oder 3-Ethoxypropyl, 2-oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxy-butyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 3,6,9,12-Tetraoxatridecyl oder 3,6,9,12-Tetraoxatetradecyl; entsprechendes C₁-C₂₀-Alkyl, dessen C₁-C₂₀-Kohlenstoffkette durch Schwefelatome in Thioetherfunktion, nichtbenachbarte Imino-, C₁-C₂₀-Alkylimino- und/oder Carbonylgruppen unterbrochen sein kann, lässt sich formal aus den zuvor exemplarisch aufgeführten Sauerstoff enthaltenden Resten durch Ersatz der Sauerstoffatome mit Schwefelatomen, nichtbenachbarten Imino-, C₁-C₂₀-Alkylimino- und/oder Carbonylgruppen herleiten.

Geeignete reaktive Reste Z¹ und Z², über welche eine Polymerisation herbeigeführt werden kann, sind beispielsweise 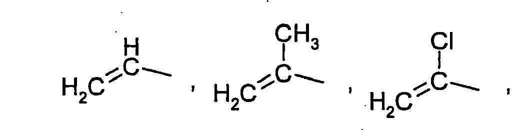 HC≡C-, HC≡C-CH₂- , 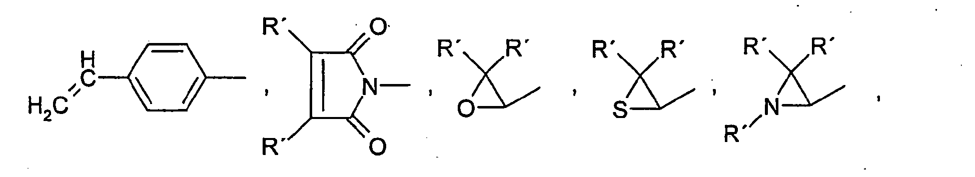 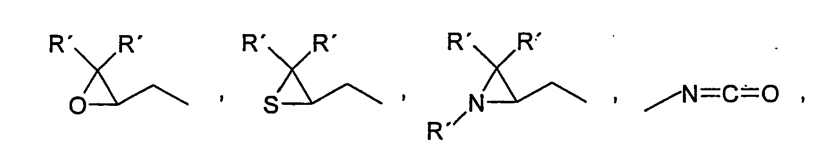 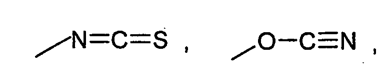 -C≡N, -COOH , -OH oder -NH₂ ,
wobei unter Polymerisation alle Aufbaureaktionen von Polymeren verstanden werden sollen, also Additionspolymerisationen als Kettenreaktionen, Additionspolymerisationen als Stufenreaktionen sowie Kondensationspolymerisationen.

Die Variablen R' der zuvor beispielhaft gezeigten reaktiven Reste stehen hierbei für Wasserstoff oder C₁-C₄-Alkyl, d.h. Methyl, Ethyl, n-Propyl, Isopropyl, n-Butyl, sec-Butyl, Isobutyl oder tert-Butyl und können gleich oder verschieden sein.

Verbindungen mit Cyanatresten können spontan zu den entsprechenden Cyanuraten, solche mit Cyanoresten, insbesondere unter Katalyse von Säuren, wie z.B. Salzsäure, oder Basen, zu den entsprechenden Triazinen trimerisieren. Verbindungen mit Epoxid-, Thiiran-, Aziridin-, Isocyanat- und Isothiocyanatgruppen benötigen zur Polymerisation üblicherweise weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Die komplementären reaktiven Gruppen können dabei entweder in einer zweiten erfindungsgemäßen Verbindung vorhanden sein, die mit der ersteren gemischt wird, oder sie können durch Hilfsverbindungen, die zwei oder mehr dieser komplementären Gruppen enthalten, in das Polymerisationsgemisch eingebracht werden. Enthalten diese Verbindungen jeweils zwei dieser reaktiven Gruppen, so entstehen lineare Polymere mit überwiegend thermoplastischem Charakter. Enthalten die Verbindungen mehr als zwei reaktive Gruppen, so entstehen vernetzte Polymere, die mechanisch besonders stabil sind. Die Maleinimidogruppe eignet sich besonders zur radikalischen Copolymerisation mit olefinischen Verbindungen wie Styrol.

Bevorzugte reaktive Reste Z¹ und Z² in den erfindungsgemäßen Verbindungen sind ausgewählt aus der Gruppe bestehend aus

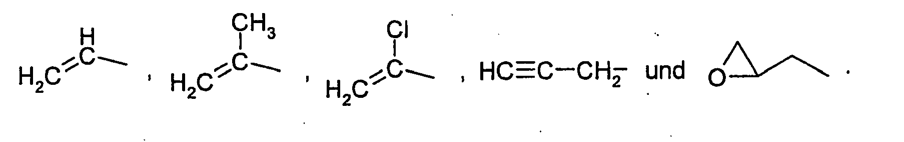

Besonders bevorzugte reaktive Reste Z¹ und Z² sind

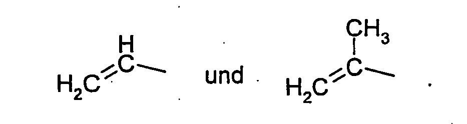

Bevorzugte reaktive Gruppierungen Z¹-Y¹ und Z²-Y² in den erfindungsgemäßen Verbindungen sind ausgewählt aus der Gruppe bestehend aus 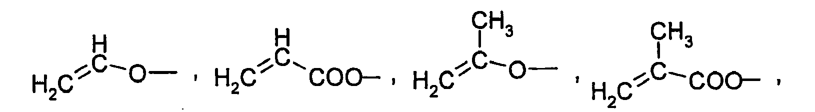 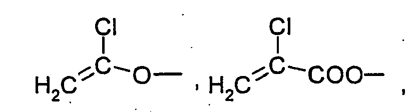 HC≡C-CH₂O-, HC≡C-CH₂COO-,

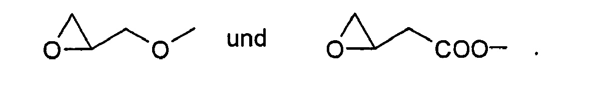

Besonders bevorzugte reaktive Gruppierungen Z¹-Y¹ und Z²-Y² sind hierbei

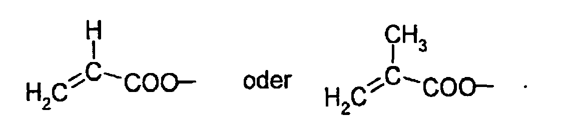

Die in den Brücken Y¹ bis Y³ aufscheinende Variable R steht neben Wasserstoff auch für C₁-C₄-Alkyl, d.h. Methyl, Ethyl, n-Propyl, Isopropyl, n-Butyl, sec-Butyl, Isobutyl oder tert-Butyl.

Als Spacer A¹ und A² kommen alle für diesen Zweck bekannten Gruppen in Betracht. Die Spacer enthalten 1 bis 30, vorzugsweise 3 bis 12 Kohlenstoffatome und bestehen aus vorwiegend linearen aliphatischen Gruppen. Sie können in der Kette z.B. durch nicht benachbarte Sauerstoff- oder Schwefelatome oder Imino- oder C₁-C₄-Alkyliminogruppen, wie Methyliminogruppen, unterbrochen sein. Als mögliche Substituenten für die Spacerkette kommen Fluor, Chlor, Brom, Cyan, Methyl und Ethyl in Betracht. Insbesondere können an Kohlenstoffatome gebundenen Wasserstoffatome der Spacerkette teilweise oder vollständig durch Fluoratome substituiert sein.

Repräsentative Spacer A¹ und A² sind beispielsweise:
-(CH₂)ₚ-, -(CH₂CH₂O)ₘCH₂CH₂-, -CH₂CH₂SCH₂CH₂-, -CH₂CH₂NHCH₂CH₂-, 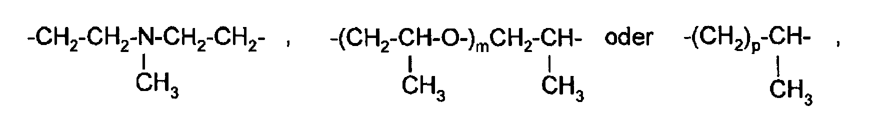 wobei m für 1 bis 3 und p für 1 bis 12 steht.

Bei den Resten T² handelt es sich um zweiwertige gesättigte oder ungesättigte iso-oder heterocyclische Reste. Diese können nicht nur aus einem, sondern auch aus mehreren, miteinander kondensierten Ringen bestehen. So kommen für T² beispielsweise auch zweiwertige Chinolin-, Decalin- oder Naphthalinreste in Betracht.

Vorzugsweise sind die Reste T² in den polymerisierbaren flüssigkristallinen Verbindungen der Formel I zweiwertige Reste ausgewählt aus der Gruppe bestehend aus 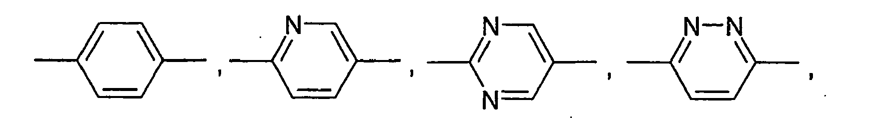 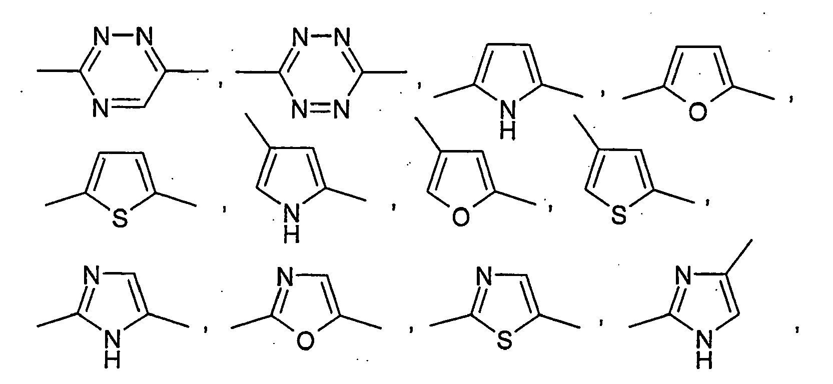 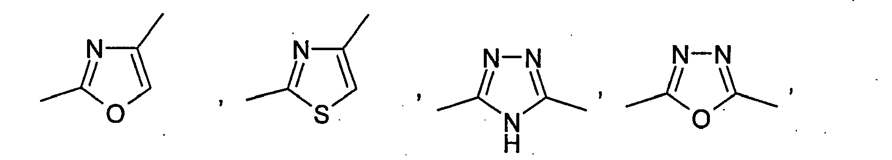 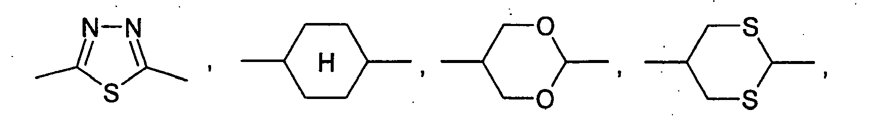 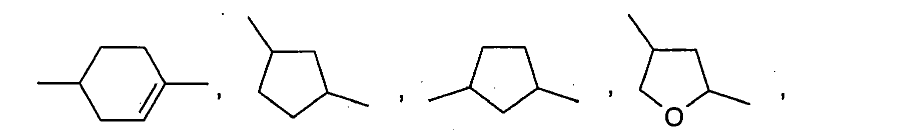 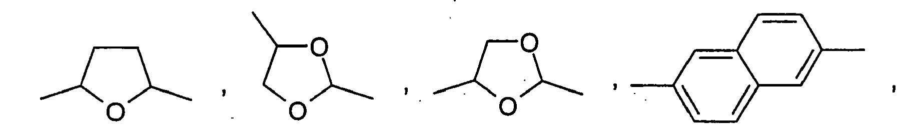 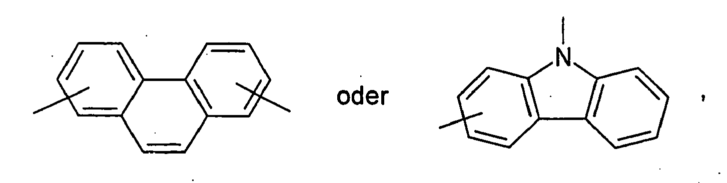 wobei die Reste 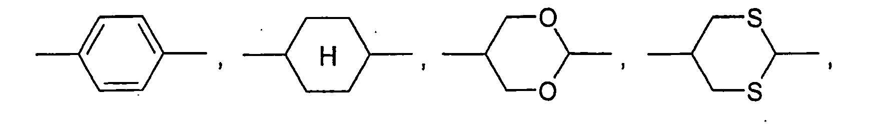 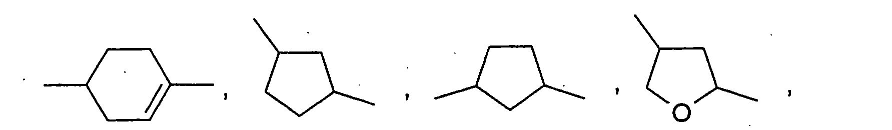 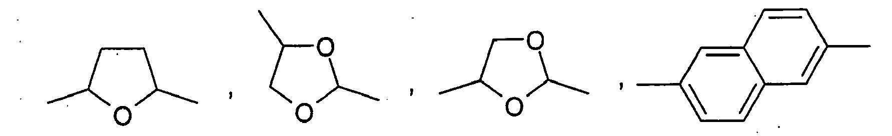 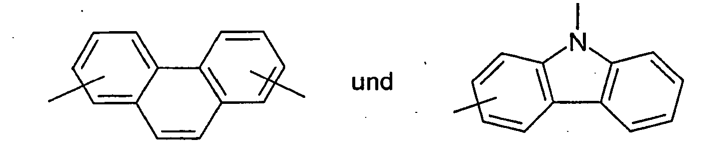 mit bis zu vier gleichen oder verschiedenen Substituenten,
der Rest 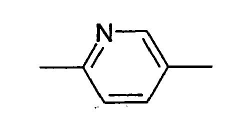 mit bis zu drei gleichen oder verschiedenen Substituenten,
die Reste 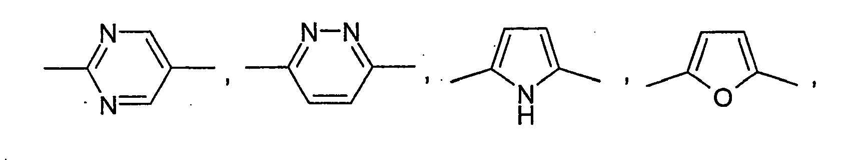 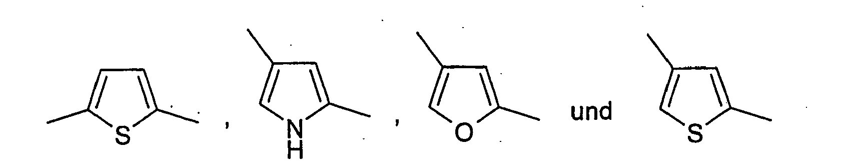 mit bis zu zwei gleichen oder verschiedenen Substituenten
und
die Reste 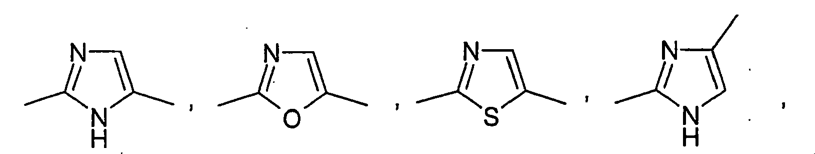 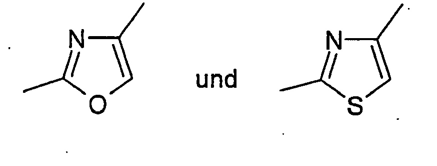 mit einem Substituenten

Halogen, NO₂, NO, CN, CHO, L¹, CO-L¹, X¹-CO-L¹, X¹-SO-L¹, X¹-SO₂-L¹, X¹-L^{1'}, CO-X¹-L¹', O-CO-X¹-L^{1'}, SO-X¹-L^{1'} oder SO₂X¹-L^{1'} substituiert sein können, wobei bedeuten
- L¹: C₁-C₂₀-Alkyl, C₂-C₂₀-Alkenyl, C₂-C₂₀-Alkinyl, C₆-C₁₀-Aryl, Heteroaryl mit 2 bis 12 Kohlenstoffatomen, C₆-C₁₀-Aryl-C₁-C₂₀-alkyl, C₆-C₁₀-Aryl-C₂-C₂₀-alkenyl, C₆-C₁₀-Aryl-C₂-C₂₀-alkinyl, Heteroaryl-C₁-C₂₀-alkyl, Heteroaryl-C₁-C₂₀-alke- nyl oder Heteroaryl-C₁-C₂₀-alkinyl mit jeweils 2 bis 12 Kohlenstoffatomen im Heteroarylrest, wobei die C₁-C₂₀-Kohlenstoffkette durch Sauerstoffatome in Etherfunktion, Schwefelatome in Thioetherfunktion, nichtbenachbarte Imino-, C₁-C₂₀-Alkylimino- und/oder Carbonylgruppen unterbrochen sein kann und sowohl das C₆-C₁₀-Aryl als auch das Heteroaryl mit einem oder mehreren Substituenten ausgewählt aus der Gruppe bestehend aus Halogen, NO₂. NO, CN, CHO, L², CO-L², X²-CO-L², X²-SO-L², X²-SO₂-L²-, X²-L^{2'}, CO-X²-L^{2'}, O-CO-X²-L^{2'}, SO-X²-L^{2'} und SO₂-X²-L^{2'} substituiert sein kann, kann,
- L^{1'}: Wasserstoff oder unabhängig von L¹ die gleiche Bedeutung wie L¹,
- L²: C₁-C₂₀-Alkyl, C₂-C₂₀-Alkenyl, C₂-C₂₀-Alkinyl, C₆-C₁₀-Aryl, Heteroaryl mit 2 bis 12 Kohlenstoffatomen, C₆-C₁₀-Aryl-C₁-C₂₀-alkyl, C₆-C₁₀-Aryl-C₂-C₂₀-alkenyl, C₆-C₁₀-Aryl-C₂-C₂₀-alkinyl, Heteroaryl-C₁-C₂₀-alkyl, Heteroaryl-C₂-C₂₀-alke- nyl oder Heteroaryl-C₂-C₂₀-alkinyl mit jeweils 2 bis 12 Kohlenstoffatomen im Heteroarylrest,
- L^{2'}: Wasserstoff oder unabhängig von L² die gleiche Bedeutung wie L²
und
- X¹, X²: unabhängig voneinander Sauerstoff, Schwefel oder NL^{1'} bzw. NL^{2'},
wobei in den Resten L¹ und/oder L² die an Kohlenstoffatome gebundenen Wasserstoffatome teilweise oder vollständig durch Halogenatome substituiert sein können.

Beispiele für die Variablen L¹ und L^{1'} in der Bedeutung eines C₁-C₂₀-Alkyl, dessen C₁-C₂₀-Kohlenstoffkette durch Sauerstoffatome in Etherfunktion, Schwefelatome in Thioetherfunktion, nichtbenachbarte Imino-, C₁-C₂₀-Alkylimino- und/oder Carbonylgruppen unterbrochen sein kann, sowie für die Variablen L² und L^{2'} in der Bedeutung eines C₁-C₂₀-Alkyl wurden bereits zuvor bei den Variablen Z¹ und Z² exemplarisch aufgeführt.

Beispiele für die Variablen L¹ und L^{1'} in der Bedeutung eines C₂-C₂₀-Alkenyl, dessen C₂-C₂₀-Kohlenstoffkette durch Sauerstoffatome in Etherfunktion, Schwefelatome in Thioetherfunktion, nichtbenachbarte Imino-, C₁-C₂₀-Alkylimino- und/oder Carbonylgruppen unterbrochen sein kann, sowie für die Variablen L² und L^{2'} in der Bedeutung eines C₂-C₂₀-Alkenyl sind insbesondere C₂-C₂₀-Alk-1-enylreste. Diese Reste lassen sich von geeigneten, zuvor bei den Variablen Z¹ und Z² exemplarisch aufgeführten Resten durch formalen Ersatz zweier Wasserstoffatome, welche sich an benachbarten Kohlenstoffatomen befinden, durch eine weitere Kohlenstoff-Kohlenstoff-Bindung ableiten.

Beispiele für die Variablen L¹ und L^{1'} in der Bedeutung eines C₂-C₂₀-Alkinyl, dessen C₂-C₂₀-Kohlenstoffkette durch Sauerstoffatome in Etherfunktion, Schwefelatome in Thioetherfunktion, nichtbenachbarte Imino-, C₁-C₂₀-Alkylimino- und/oder Carbonylgruppen unterbrochen sein kann, sowie für die Variablen L² und L^{2'} in der Bedeutung eines C₂-C₂₀-Alkinyl sind insbesondere C₂-C₂₀-Alk-1-inylreste. Diese Reste lassen sich von geeigneten, zuvor bei den Variablen Z¹ und Z² exemplarisch aufgeführten Resten durch formalen Ersatz von vier Wasserstoffatomen, welche sich an benachbarten Kohlenstoffatomen befinden, durch zwei weitere Kohlenstoff-Kohlenstoff-Bindungen ableiten.

Beispiele für die Variablen L¹ und L^{1'}, in der Bedeutung von C₆-C₁₀-Aryl-C₁-C₂₀-alkyl, C₆-C₁₀-Aryl-C₂-C₂₀-alkenyl, C₆-C₁₀-Aryl-C₂-C₂₀-alkinyl, Heteroaryl-C₁-C₂₀-alkyl, Heteroaryl-C₂-C₂₀-alkenyl und Heteroaryl-C₂-C₂₀-alkinyl mit jeweils 2 bis 12 Kohlenstoffatomen im Heteroarylrest, wobei die C₁-C₂₀-Kohtenstoffkette durch Sauerstoffatome in Etherfunktion, Schwefelatome in Thioetherfunktion, nichtbenachbarte Imino-, C₁-C₂₀-Alkylimino- und/oder Carbonylgruppen unterbrochen sein kann, sowie für die Variablen L² und L^{2'} in der Bedeutung von C₆-C₁₀-Aryl-C₁-C₂₀-alkyl, C₆-C₁₀-Aryl-C₂-C₂₀-alkenyl, C₆-C₁₀-Aryl-C₂-C₂₀-alkinyl, Heteroaryl-C₁-C₂₀-alkyl, Hetero-aryl-C₂-C₂₀-alkenyl und Heteroaryl-C₂-C₂₀-alkinyl mit jeweils 2 bis 12 Kohlenstoffatomen im Heteroarylrest sind insbesondere solche Reste, welche sich von den zuvor bei den Variablen Z¹ und Z² exemplarisch aufgeführten Resten durch formalen Ersatz eines terminalen Wasserstoffatoms durch C₆-C₁₀-Aryl bzw. Heteroaryl mit 2 bis 12 Kohlenstoffatomen ableiten lassen.

Für die Variablen L¹ und L^{1'}, sowie L² und L^{2'} kommen als C₈-C₁₀-Aryl insbesondere Phenyl und Naphthyl in Frage.

Für die Variablen L¹ und L^{1'}, sowie L² und L^{2'} kommen als Heteroaryl mit 2 bis 12 Kohlenstoffatomen solche Reste in Frage, welche sich z.B. von Pyrrol, Furan, Thiophen, Pyrazol, Isoxazol, Isothiazol, Imidazol, 1H-1,2,3-Triazol, 1 H-1,2,4-Triazol, Pyridin, Pyrazin, Pyridazin, 1H-Azepin, 2H-Azepin, Oxazol, Thiazol, 1,2,3-, 1,2,4- oder 1,3,4-Oxadiazol, 1,2,3-, 1,2,4- oder 1,3,4-Thiadiazol sowie gegebenenfalls den benzo-oder dibenzoanellierten Ringen, wie z.B. Chinolin, Isochinolin, Indol, Benzo[b]furan (Cumaron), Benzo[b]thiophen (Thionaphthen), Carbazol, Dibenzofuran, Dibenzothiophen, 1H-Indazol, Indoxazol, Benzo[d]isothiazol, Anthranil, Benzimidazol, Benzoxazol, Benzothiazol, Cinnolin, Phthalazin, Chinazolin, Chinoxalin oder Phenazin ableiten.

Im Falle der Variablen L¹ und L^{1'}, kann das C₈-C₁₀-Aryl bzw. Heteroaryl mit 2 bis 12 Kohlenstoffatomen mit einem oder mehreren Substituenten Halogen, NO₂, NO, CN, CHO, L², CO-L², X²-CO-L², X²-SO-L², X²-SO₂-L², X²-L^{2'}, CO-X²-L^{2'}, O-CO-X²-L^{2'}, SO-X²-L^{2'} und SO₂-X²-L^{2'} substituiert sein.

Halogen bedeutet Fluor, Chlor, Brom oder Iod, insbesondere Fluor oder Chlor.

Die Variablen X¹ und X² bedeuten unabhängig voneinander Sauerstoff, Schwefel oder NL^{1'} bzw. NL^{2'}.

In den Resten L¹ und/oder L² können die an Kohlenstoffatome gebundenen Wasserstoffatome teilweise oder vollständig durch Halogenatome substituiert sein. Als solche kommen hier Chloratome und insbesondere Fluoratome in Betracht.

Vorteilhafte erfindungsgemäße Verbindungen entsprechen der nachfolgend gezeigten Formel I 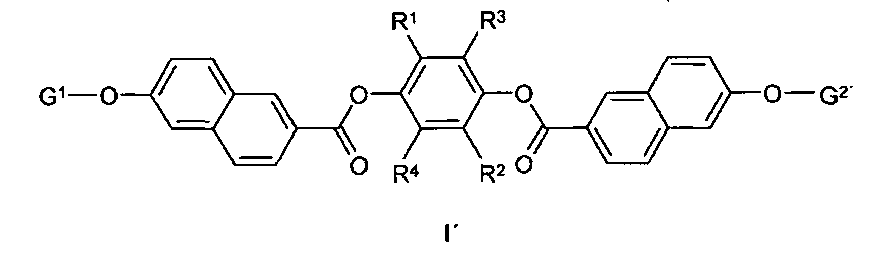 in welcher G¹ und G^{2'} eine Gruppierung Z¹-Y¹-A¹-O-COO- bzw. -COO-O-A²Y²-Z² bedeuten und R¹ bis R⁴ unabhängig voneinander für Wasserstoff oder Substituenten ausgewählt aus der Gruppe bestehend aus Halogen, NO₂, CN, CHO, L¹ CO-L¹, X¹-CO-L¹, X¹-L^{1'} und CO-X¹-L^{1'} stehen, wobei bedeuten
- L¹: C₁-C₂₀-Rkyl,
- L^{1'}: Wasserstoff oder C₁-C₂₀-Alkyl,
und X¹ Sauerstoff oder NL^{1'}.

In besonders vorteilhaften Verbindungen der zuvor gezeigten Formel I' sind die Gruppierungen G¹ und G^{2'} gleich.

In weiteren besonders vorteilhaften Verbindungen der zuvor gezeigten Formel I' bedeuten entweder die beiden Substituenten R¹ und R³ oder die beiden Substituenten R⁴ und R² Wasserstoff.

In weiteren besonders vorteilhaften Verbindungen der zuvor gezeigten Formel I' bedeutet mindestens einer der beiden Substituenten R¹ oder R³ Halogen, NO₂, CN, CHO, L¹, CO-L¹, X¹-CO-L¹, X¹-L¹- oder CO-X¹-L^{1'} und die beiden Substituenten R⁴ und R² sowie gegebenenfalls einer der Substituenten R¹ oder R³ bedeuten Wasserstoff, wobei L¹ für C₁-C₂₀-Alkyl, L^{1'} für Wasserstoff oder C₁-C₂₀-Alkyl und X¹ für Sauerstoff oder NL^{1'}, steht.

In weiteren besonders vorteilhaften Verbindungen der zuvor gezeigten Formel I' bedeutet mindestens einer der beiden Substituenten R¹ oder R³ L¹, CO-L¹, X¹-CO-L¹, X¹-L¹ oder CO-X¹-L^{1'} und die beiden Substituenten R⁴ und R² sowie gegebenenfalls einer der Substituenten R¹ oder R³ bedeuten Wasserstoff, wobei L¹ für C₁-C₂₀-Alkyl, L^{1'} für Wasserstoff oder C₁-C₂₀-Alkyl und X¹ für Sauerstoff oder NL^{1'} steht.

In weiteren besonders vorteilhaften Verbindungen der zuvor gezeigten Formel bedeutet mindestens einer der beiden Substituenten R¹ oder R³ L¹, X¹-L¹ oder CO-X¹-L¹ und die beiden Substituenten R⁴ und R² sowie gegebenenfalls einer der Substituenten R¹ oder R³ bedeuten Wasserstoff, wobei L¹ für C₁-C₂₀-Alkyl und X¹ für Sauerstoff steht.

Weiterer Gegenstand der vorliegenden Erfindung ist eine polymerisierbare oder nicht polymerisierbare flüssigkristalline Zusammensetzung, welche als Komponenten 10 bis 100 Gew.% der erfindungsgemäßen Verbindungen der Formel I oder deren bevorzugten Ausführungsformen, 0 bis 90 Gew.-% weitere Monomere, 0 bis 50 Gew.-% einer oder mehrerer chiraler Verbindungen und 0 bis 90 Gew.-% weitere Zusätze enthält, wobei die Summe der Anteile der Komponenten sich zu 100 Gew.-% ergänzt.

Unter einer polymerisierbaren oder nicht polymerisierbaren erfindungsgemäßen flüssigkristallinen Zusammensetzung ist dabei ganz allgemein nicht nur eine solche Zusammensetzung zu verstehen, deren eine oder mehrere Komponenten bereits per se (im interessierenden Temperaturbereich) flüssigkristalline Eigenschaften besitzen, vielmehr ist hierunter auch eine solche Zusammensetzung zu verstehen, die erst durch Mischen der Bestandteile oder auch erst durch Zumischen der erfindungsgemäßen Verbindungen flüssigkristallines Verhalten aufweist (z.B. Iyotrope Systeme). Desweiteren können die erfindungsgemäßen Verbindungen der Formel I und deren bevorzugte Ausführungsformen bereits selbst flüssigkristallines Verhalten aufweisen, sie müssen diese Eigenschaft aber nicht notwendigerweise besitzen.

Eine nicht polymerisierbare erfindungsgemäßenZusammensetzung ist insbesondere eine solche Zusammensetzung, welche unter üblichen Polymerisationsbedingungen nicht zur Ausbildung von selbsttragenden Polymerisations- oder Kondensationsprodukten befähigt ist. Diese Zusammensetzung lässt sich beispielsweise durch Vermischen geeigneter, kommerziell erhältlicher flüssigkristalliner Materialien, wie sie zum Beispiel für aktive LC-Schichten in der Displaytechnik Verwendung finden, mit einer oder mehreren der erfindungsgemäßen Verbindungen der Formel I oder deren bevorzugten Ausführungsformen herstellen. Handelt es sich bei letzteren um Verbindungen, welche ein oder zwei reaktive Reste Z¹ und Z² enthalten, so sind diese in den erfindungsgemäßen Zusammensetzungen in einer solchen Konzentration vorhanden, dass diese zur Erzeugung entsprechend dicht vernetzter selbsttragender Polymerisations- oder Kondensationsprodukte nicht ausreichend ist.

Eine polymerisierbare erfindungsgemäße Zusammensetzung ist insbesondere eine solche Zusammensetzung, in welcher mindestens eine der Komponenten unter üblichen Polymerisationsbedingungen.zur Ausbildung von Polymerisations- oder Kondensationsprodukten befähigt ist und welche als solche polymerisierbar ist und zu selbsttragenden Produkten polymerisiert bzw. kondensiert werden kann.

Je nach Anzahl der reaktiven Reste in den Komponenten der polymerisierbaren flüssigkristallinen Zusammensetzung, lässt sich der gewünschte Polymerisations-, Vernetzungs- und/oder Kondensationsgrad nach erfolgter Polymerisation oder Kondensation einstellen. Diese Zusammensetzung lässt sich leicht durch Vermischen geeigneter Materialien mit einer oder mehreren der erfindungsgemäßen Verbindungen herstellen, wobei diese Materialien selbst polymerisierbar oder nicht polymerisierbar, flüssigkristallin oder auch nicht flüssigkristallin sein können. Geeignete polymerisierbare, flüssigkristalline Materialien sind Verbindungen, welche beispielsweise in den WO-Schriften 95/22586 A1, 95/24454 A1, 95/24455 A1, 96/04351 A1, 96124647 A1, 97/00600 A2, 97/34862 A1 und 98/47979 A1 sowie den Schriften EP 1 134 270 A1 und DE 198 35 730 A1 beschrieben sind und im Wesentlichen dem schematischen Aufbau P-Y-A-Y-M-Y-A-Y-P entsprechen, worin die Variablen P, Y und A analoge Bedeutungen wie die Variablen Z¹ und Z², Y¹ bis Y³ und A¹ und A² in Formel besitzen, und M eine mesogene Einheit bezeichnet.

Die reaktiven Verbindungen, welche in der Schrift DE 100 25 782 A1 als Bestandteil B) der dort beschriebenen flüssigkristallinen Stoffgemenge aufgeführt sind, können der erfindungsgemäßen flüssigkristallinen Zusammensetzung als weitere Monomere beigemengt werden. Diese meist kostengünstigen Verbindungen zeigen selbst in der Regel kein flüssigkristallines Verhalten, ihre Zumischung eröffnet jedoch die Möglichkeit, den Anteil an kostspieligen Komponenten in der erfindungsgemäßen Zusammensetzung zu reduzieren ohne deren flüssigkristallines Verhalten merklich zu beeinflussen. Darüberhinaus lassen sich mit Hilfe solcher reaktiver Monomerer gezielt Eigenschaften der Zusammensetzung, wie Vernetzungsgrad, Viskosität, Elastizität usw. einstellen. Die Auswahl geeigneter reaktiver Monomerer ist vom Fachmann, gegebenenfalls nach Durchführung von Vorversuchen, leicht zu bewerkstelligen. Anzumerken ist hier, dass solche reaktiven Verbindungen auch als (Hilfs-) Verbindungen im weiter oben diskutierten Sinne wirken können.

Je nach beabsichtigter Verwendung der erfindungsgemäßen flüssigkristallinen Zusammensetzung kann diese noch mindestens eine chirale Verbindung enthalten. Durch deren Zugabe erhält man eine chiral-nematische bzw. cholesterische flüssigkristalline Zusammensetzung, welche besondere optische Eigenschaften, wie etwa vom Blickwinkel abhängige Farbeffekte, Reflektion im IR- oder UV-Wellenlängenbereich des Spektralbereichs usw., besitzen.

Bevorzugte chirale Verbindungen entsprechen hierbei den allgemeinen Formeln (P-Y-)ₚX, (P-Y-A-Y-)ₚX und (P-Y-A-Y-M-Y-)ₚ-X, in welchen die Definitionen der Variablen P, Y und A denen der Variablen Z¹ und Z², Y¹ bis Y³ und A¹ und A² von Formel I entsprechen, M eine mesogene Gruppe bezeichnet, p für Werte von 1, 2, 3, 4, 5 oder 6 und X für entsprechende p-wertige chirale Reste steht, wobei die p an den chiralen Rest X gebundenen Gruppierungen gleich oder verschieden sein können.

Mögliche Reste X sind beispielsweise auf den Seiten 5 bis 9 der Schrift WO 95/16007 A1 gezeigt, wobei insbesondere die zweiwertigen Reste 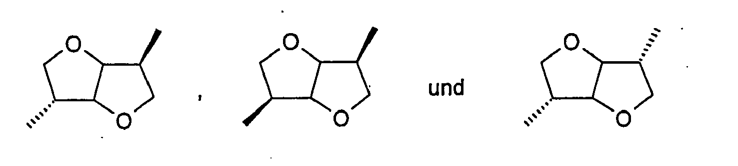 zu nennen sind.

Weitere chirale Verbindungen, welche die genannten sowie andere geeignete chirale Reste X enthalten, sind beispielsweise in den Schriften EP 0 747 382 A1, EP 0 750 029 A1, EP 1 136 478 A1 und DE 198 43 724 A1 genannt.

Die erfindungsgemäße flüssigkristalline Zusammensetzung kann noch weitere Zusätze enthalten. Als solche kommen in Frage Photoinitiatoren, Verdünnungsmittel, Verdickungsmittel, Entschäumer und Entlüfter, Gleit- und Verlaufshilfsmittel, thermisch härtende oder strahlenhärtende Hilfsmittel, Substratnetzhilfsmittel, Netz- und Dispergierhilfsmittel, Hydrophobierungsmittel, Haftvermittler und Hilfsmittel zur Verbesserung der Kratzfestigkeit, Farbstoffe und Pigmente sowie Zusätze ausgewählt aus der Gruppe der Licht-, Hitze- und/oder Oxidationsstabilisatoren. Auf die chemisch-physikalische Natur dieser Zusätze wird ausführlich in der Schrift WO 00/47694 A1 eingegangen.

Weiterer Gegenstand der vorliegenden Erfindung ist ein Oligomer oder Polymer, welches durch Oligomerisation oder Polymerisation einer erfindungsgemäßen polymerisierbaren flüssigkristallinen Zusammensetzung erhältlich ist. Dieses erfindungsgemäße Oligomer oder Polymer kann insbesondere auch in Form eines Filmes, d.h. einer selbsttragenden Schicht gleichförmiger Dicke, vorliegen. Dieser Film kann sich auf einem Substrat befinden, welches so beschaffen ist, dass durch geeignete Maßnahmen eine leichte Ablösung und Übertragung auf ein anderes Substrat zum permanenten Verbleib möglich ist. Solch ein Film ist beispielsweise im Bereich der Folienbeschichtung und in Kaschierverfahren verwendbar.

Desweiteren lassen sich solche Filme -in ihren Eigenschaften dem entsprechenden Verwendungszweck angepasst- in verschiedensten Bereichen einsetzen. Beispielsweise können sie in Vorrichtungen zur Darstellung optischer Information Verwendung finden. Solche Vorrichtungen sind etwa Video- oder Overheadprojektoren, elektrophoretische Anzeigevorrichtungen, Verkehrsanzeigen, LCDs zur Verwendung in Computermonitoren, Fernsehern, Bildschirmen in Druckern oder Küchengeräten sowie Reklametafeln, Beleuchtungen und Hinweistafeln desweiteren mobile Bildschirme wie z.B. Bildschirme in Handys, Laptops, Digitalkameras, Fahrzeugen sowie Zielanzeigen an Bussen und Bahnen. Sie können in diesen Vorrichtungen in verschiedenster Funktion enthalten sein, beispielsweise als Farbfilter oder Folien für die Erzeugung von wellenlängen-selektivem oder breitbandig polarisiertem Licht.

Weiterer Gegenstand der vorliegenden Erfindung ist ein Verfahren zum Bedrucken oder Beschichten eines Substrats, welches dadurch gekennzeichnet ist, dass man eine erfindungsgemäße polymerisierbare flüssigkristalline Zusammensetzung auf das Substrat aufbringt und anschließend polymerisiert.

Hinsichtlich der Verfahrensweise zum Bedrucken oder Beschichten von Substraten mit flüssigkristallinen Materialien sei sinngemäß auf die Schrift WO 96/02597 A2 verwiesen. Darüberhinaus ist auch eine mit Hilfe des erfindungsgemäßen Verfahrens hergestellte, und die ursprüngliche Substratoberfläche teilweise oder vollständig bedeckende polymerisierte Schicht als Substrat im Sinne der Anmeldung zu verstehen, so dass erfindungsgemäß auch die Herstellung von mehrfach bedruckten und/oder beschichteten Substraten umfasst ist.

Anzumerken ist hier weiter, dass unter "Bedrucken" üblicherweise das unvollständige Bedecken der Substratoberfläche, unter "Beschichten" das vollständige Bedecken der Substratoberfläche verstanden wird.

Als Substrate kommen desweiteren neben Papier- und Kartonageprodukten, beispielsweise für Tragetaschen, Zeitschriften, Broschüren, Geschenkverpackungen und Verpackungsmaterialien für Gebrauchs-, Genuss- und Luxusgüter, auch Folien, etwa für dekorative und nicht-dekorative Verpackungszwecke, sowie Textilien jedweder Art und Leder in Frage. Desweiteren kommen als Substrate auch solche zur Herstellung von Banknoten, Wertpapieren, Eintrittskarten u.ä. verwendete Materialien in Betracht.

Weitere Substrate sind aber auch Güter der (Unterhaltungs-)Elektronik, wie etwa Musikcassetten (MCs), SVHS- und VHS-Cassetten, Minidisks (MDs), Compactdisks (CDs), Digital Versatile Disks (DVDs) und die entsprechenden Wiedergabe- und/oder Aufnahmegeräte, Fernseher, Radios, Telefone/Handys, EDV-Geräte usw. und Güter aus dem Freizeit-, Sport-, Haushalts- und Spielsektor, wie etwa Fahrräder, Kinderfahrzeuge, Skier, Snow- und Surfboards, Inline-Skater und Roll- und Schlittschuhe sowie Haushaltsgeräte. Darüber hinaus sind unter solchen Substraten beispielsweise auch Schreibutensilien und Brillengestelle zu verstehen.

Weitere Substrate sind aber auch verschiedenste Folien, welche in optischen oder elektrooptischen Bauteilen oder bei deren Herstellung Verwendung finden. Solche Folien bestehen beispielsweise aus Polyvinylalkohol (PVA), Triacetylcellulose (TAC), Polyimid (PI), Polyvinylcinnamat (PVC) oder Polyolefinen, wie etwa Polynorbornen, und können beispielsweise (Breitband-)Polarisatoren, lichtleitende Elemente für die Hintergrundbeleuchtung in LCDs (sogenannte "light guides"), Folien für die Verteilung des Lichts (sogenannte "BEFs", d.h. "brightness enhancement films") und Folien für die Erzeugung von polarisiertem Licht in LCDs (sogenannte "DBEFs", d.h. "dual brightness enhancement films") sein. Weitere Substrate in diesem Zusammenhang können aber auch bestimmte Baugruppen der LCDs sein, wie etwa Glas- oder Polymerscheiben, welche gegebenenfalls noch eine transparente leitfähige Beschichtung, beispielsweise aus Indium-Zinn-Oxid (ITO), besitzen.

Light guides oder BEFs können beispielsweise direkt mit der erfindungsgemäßen nematischen (d.h. der Anteil der chiralen Verbindung(en) beträgt 0 Gew.-%) oder chiral-nematischen Zusammensetzung beschichtet und diese anschließend polymerisiert werden. Der Beschichtungvorgang kann hierbei mehr oder weniger oft mit erfindungsgemäßen Zusammensetzungen gleicher oder unterschiedlicher Zusammensetzung wiederholt werden, um entsprechende optische Komponenten, wie etwa Verzögerungsfilme (sogenannte "retardation films"), (Breitband-)Polarisatoren und optische Filter zu erzeugen. Hierdurch lässt sich ein entsprechend kompakterer Aufbau der optischen Bauteile in LCDs herstellen.

Weiter kann durch das erfindungsgemäße Verfahren eine geeignete nematische Schicht als Verzögerungsfilm auf einen, zirkular polarisiertes Licht liefernden (Breitband-)Polarisator aufgebracht werden. Hierdurch kann zirkular polarisiertes Licht in linear polarisiertes überführt werden. Der (Breitband-)Polarisator kann hierbei ebenfalls aus erfindungsgemäßen Zusammensetzungen, gegebenenfalls unter Verwendung des erfindungsgemäßen Verfahrens, hergestellt worden sein.

Weitere Substrate sind aber auch im Bausektor anzutreffende Oberflächen, wie Gebäudewände oder auch Fensterscheiben. In letzterem Fall kann neben einem dekorativen auch ein funktioneller Effekt gewünscht sein. So ist es möglich, Mehrfachschichten auf dem Fenstermaterial zu erzeugen, deren einzelne Schichten verschiedene chemisch-physikalische Eigenschaften besitzen. Werden etwa unter Zugabe eines Enantiomeren einer chiralen Verbindung sowie des entsprechenden optischen Antipoden einzelne Schichten der polymerisierten flüssigkristallinen Zusammensetzung mit entgegengesetzter Verdrillung oder werden unter Zugabe unterschiedlicher Konzentrationen an chiraler Verbindung einzelne Schichten der polymerisierten flüssigkristallinen Zusammensetzung gleichen Drehsinns aber jeweils unterschiedlicher Ganghöhe und somit unterschiedlicher Reflektionseigenschaften aufgebracht, so können gezielt bestimmte Wellenlängen oder Wellenlängenbereiche des Lichtspektrums reflektiert werden. Hierdurch ist beispielsweise eine IR- oder UV-reflektierende Fensterbeschichtung möglich.

Dementsprechend ist weiterer Gegenstand der voliegenden Erfindung die Verwendung der erfindungsgemäßen flüssigkristallinen Zusammensetzung oder der erfindungsgemäßen Oligomeren oder Polymeren zur Herstellung einer Wärmeisolationsbeschichtung, umfassend eine oder mehrere cholesterische Schichten, die im infraroten Wellenlängenbereich, vorzugsweise oberhalb 750 nm, insbesondere im Wellenlängenbereich .von 751 nm bis etwa 2000 nm mindestens 40 %, insbesondere mindestens 45 % der auftreffenden Strahlung reflektiert.

Insbesondere ist weiterer Gegenstand der vorliegenden Erfindung die Verwendung der erfindungsgemäßen flüssigkristallinen Zusammensetzung oder der erfindungsgemäßen Oligomeren oder Polymeren zur Herstellung einer Wärmeisolationsbeschichtung, umfassend eine oder mehrere cholesterische Schichten, die im infraroten Wellenlängenbereich, vorzusweise oberhalb 750 nm, insbesondere im Wellenlängenbereich von 751 nm bis etwa 2000 nm mindestens 40 %, insbesondere mindestens 45 % der auftreffenden Strahlung reflektiert und die im Wellenlängenbereich von etwa 390 nm bis 750 nm eine Transmission von mindestens 80%, insbesondere mindestens 90 % der auftreffenden Strahlung aufweist.

Weiterer Gegenstand der vorliegenden Erfindung ist eine Wärmeisolationsbeschichtung, umfassend eine oder mehrere cholesterische Schichten, die im infraroten Wellenlängenbereich, vorzugsweise oberhalb 750 nm, insbesondere im Wellenlängenbereich von 751 nm bis etwa 2000 nm mindestens 40 %, insbesondere mindestens 45 % der auftreffenden Strahlung reflektiert, und welche unter Verwendung der erfindungsgemäßen flüssigkristallinen Zusammensetzung oder der erfindungsgemäßen Oligomeren oder Polymeren erhältlich ist.

Insbesondere ist weiterer Gegenstand der vorliegenden Erfindung eine Wärmeisolationsbeschichtung, umfassend eine oder mehrere cholesterische Schichten, die im infraroten Wellenlängenbereich, vorzugsweise oberhalb 750 nm, insbesondere im Wellenlängenbereich von 751 nm bis etwa 2000 nm mindestens 40 %, insbesondere mindestens 45 % der auftreffenden Strahlung reflektiert und die im Wellenlängenbereich von etwa 390 nm bis 750 nm eine Transmission von mindestens 80 %, insbesondere mindestens 90 % der auftreffenden Strahlung aufweist, und welche unter Verwendung der erfindungsgemäßen flüssigkristallinen Zusammensetzung oder der erfindungsgemäßen Oligomeren oder Polymeren erhältlich ist.

Zu diesem Aspekt der erfindungsgemäßen flüssigkristal-linen Zusammensetzung, speziell im Hinblick auf Wärmeisolationsbeschichtungen, sei sinngemäß auch auf die Schrift WO 99/19267 A1 verwiesen.

Weiterer Gegenstand der vorliegenden Erfindung ist die Verwendung der erfindungsgemäßen flüssigkristallinen Zusammensetzung oder des erfindungsgemäßen Oligomeren oder Polymeren zur Herstellung optischer Bauteile. Als solche sind z.B. LCDs sowie deren Komponenten, beispielsweise (Breitband-)Polarisatoren, optische Filter, Verzögerungsfilme (sogenannte "retardation films") und BEFs zu nennen.

Hinsichtlich der Herstellung solcher Komponenten basierend auf polymerisierbaren flüssigkristallinen Materialien sei sinngemäß etwa auf die Schrift WO 00/37585 A1 verwiesen.

Im Hinblick auf die Verwendung von erfindungsgemäßen polymerisierbaren chiral-nematischen Zusammensetzungen zur Herstellung von (Breitband-)Polarisatoren sei sinngemäß beispielsweise auf die Schriften US 6,421,107, US 6,417,902, US 6,061,108, US 6,099,758, US 6,016,177, US 5,948,831, US 5,793,456, US 5,691,789 und US 5,506,704 verwiesen.

Im Hinblick auf die Verwendung von erfindungsgemäßen polymerisierbaren nematischen Zusammensetzungen zur Herstellung von DBEFs sei sinngemäß beispielsweise auf die Schriften US 4,525,413, US 5,828,488 und US 5,965,247 verwiesen.

Die beiden letzteren Schriften beschreiben Laminate von Polymerschichten, welche in ihren optischen Eigenschaften so beschaffen sind, dass eine Schicht S¹ entweder isotropes oder anisotropes, die benachbarte Schicht S² dagegen von S¹ abweichendes anisotropes optisches Verhalten zeigt. Der Brechungsindex n₁¹ in der einen Ebenenrichtung von S¹ entspricht dabei in seinem Wert im Wesentlichen dem Brechungsindex n₁² in derselben Ebenenrichtung von S², die Brechungsindices n₂¹ bzw. n₂² in der jeweils senkrecht dazu stehenden Ebenenrichtung von S¹ bzw. S² differieren hingegen. Lichtstrahlen, welche auf diese Laminate auftreffen werden daher je nach ihrer Polarisationsrichtung entweder transmittiert (wenn n₁¹ und n₁² im Wesentlichen gleich sind) oder reflektiert (wenn n₂¹ und n₂² voneinander verschieden sind). Die anisotropen Schichten S² können daher -im Wechsel mit Polymerschichten mit geeignetem isotropem bzw. anisotropem Brechungsindex- aus entsprechenden, durch Polymerisation der erfindungsgemäßen polymerisierbaren nematischen Zusammensetzungen erhaltenen Polymeren oder Oligomeren bestehen. Als Polymerschichten S¹ kommen beispielsweise auch Klebstoffe in Frage, welche die anisotropen Schichten S² miteinander verkleben, oder Kunststoff-Folien mit geeigneter Glasübergangstemperatur, welche bei entsprechender Temperaturbehandlung zusammen mit den Schichten S² das gewünschte Laminat bilden. Zudem kann man eine Polymerschicht S¹ mit einer erfindungsgemäßen polymerisierbaren nematischen Zusammensetzung beschichten, eine weitere Polymerschicht S¹ aufbringen und dann die zwischen beiden Polymerschichten S¹ befindliche Zusammensetzung polymerisieren. Unabhängig von der gewählten Verfahrensweise zur Herstellung solcher Laminate muss natürlich eine hinreichend gute Adhäsion der Schichten S¹ und S² miteinander gewährleistet sein.

Die erfindungsgemäße flüssigkristalline Zusammensetzung lässt sich aber auch als disperse flüssigkristalline Phase in sogenannten "polymer dispersed liquid crystals" ("PDLCs") verwenden. Solche PDLCs können grundsätzlich entweder eine isotrope Polymermatrix und sowohl eine makroskopisch-isotrope als auch anisotrope disperse flüssigkristalline Phase oder eine anisotrope Polymermatrix und sowohl eine makroskopisch-isotrope als auch anisotrope disperse flüssigkristalline Phase aufweisen, wobei die makroskopisch-isotrope Phase aus der statistischen Verteilung mikroskopischanisotroper Domänen resultiert.

In der Regel wird zur Herstellung solcher PDLCs von einer (in der Regel optisch anisotropen) Polymerfolie ausgegangen, in welcher die flüssigkristalline Phase in Form feinster Einschlüsse, üblicherweise im Mikrometer- oder Submikrometergrößenbereich, gleichmäßig dispergiert vorliegt. Durch Verstrecken der Polymerfolie wird sowohl der Polymermatrix als auch der dispergierten Phase ein anisotropes optisches Verhalten aufgezwungen. Finden erfindungsgemäße polymerisierbare flüssigkristalline Zusammensetzungen Verwendung, so lässt sich der anisotrope Zustand der dispergierten Phase durch Polymerisation einfrieren und damit beispielsweise eine deutlich bessere Temperatur(wechsel)beständigkeit erzielen. Als Polymermatrix findet hierbei meist Polyvinylalkohol Verwendung.

Desweiteren kann die erfindungsgemäße polymerisierbare chiral-nematische Zusammensetzung beispielsweise auch zur Herstellung von optischen Bauteilen verwendet werden, wie sie in den Schriften US 5,235,443 und US 5,050,966 beschrieben werden.

Die erfindungsgemäße flüssigkristalline Zusammensetzung kann desweiteren auch als flüssigkristallines Farbmittel oder zur Herstellung flüssigkristalliner Farbmittel Verwendung finden. Die Verwendung als Farbmittel ist möglich, wenn die Zusammensetzung per se bereits Farbigkeit besitzt. Diese Farbigkeit kann dabei auf Interferenzeffekten der vorliegenden chiral-nematischen Phase und/oder auf Absorptionseffekten enthaltener Farbstoffe und/oder Pigmente beruhen. Desweiteren kann die Zusammensetzung -unabhängig davon ob sie Farbigkeit aufweist oder nicht- auch zur Herstellung von Farbmitteln dienen. Hinsichtlich der Herstellung von flüssigkristallinen Farbmitteln und deren Verwendung zum Bedrucken oder Beschichten von Substraten sei sinngemäß auf die Schrift WO 96/02597 A2 verwiesen.

Die erfindungsgemäße Zusammensetzung kann desweiteren in der Herstellung von Dispersionen und Emulsionen, welche bevorzugt auf Wasser basieren, Verwendung finden. Zur Herstellung solcher Dispersionen und Emulsionen unter Verwendung von flüssigkristallinen Materialien sei hierbei auf die WO-Schriften 96/02597 A2 und 98/47979 A1 verwiesen. Diese Dispersionen und Emulsionen können ebenfalls zum Bedrucken und Beschichten von Substraten, wie sie beispielhaft zuvor bereits beschrieben wurden, verwendet werden.

Weiter kann die erfindungsgemäße Zusammensetzung auch Verwendung bei der Herstellung von Pigmenten finden. Die Herstellung solcher Pigmente ist bekannt und beispielsweise ausführlich in der Schrift WO 99/11733 A1 beschrieben. Darüber hinaus lassen sich aber auch in Form und Größe voreingestellte Pigmente unter Verwendung von Drucktechniken oder mit Hilfe von Netzen, in deren Zwischenräumen sich die polymerisierbare Zusammensetzung befindet, herstellen. Der nachfolgenden Polymerisation oder Kondensation der Flüssigkristallzusammensetzung schließt sich hierbei das Ab- bzw. Herauslösen vom Substrat bzw. aus dem Netz an. Diese Vorgehensweisen sind in den WO-Schriften 96/02597 A1, 97/27251 A1, 97/27252 A1 und der Schrift EP 0 931 110 A1 detailliert beschrieben.

Diese Pigmente können einschichtig sein oder einen Mehrschichtaufbau aufweisen. Letztere Pigmente sind üblicherweise nur herstellbar, wenn Beschichtungsverfahren zur Anwendung kommen, in welchen sukzessive mehrere Schichten übereinander erzeugt und abschließend einer mechanischen Zerkleinerung unterworfen werden.

Für die zuvor beschriebene Verwendung der erfindungsgemäßen flüssigkristallinen Zusammensetzung als flüssigkristallines Farbmittel oder zur Herstellung flüssigkristalliner Farbmittel, in der Herstellung von Dispersionen und Emulsionen sowie bei der Herstellung von Pigmenten wird vorzugsweise von einer polymerisierbaren Zusammensetzung Gebrauch gemacht.

Im Rahmen der vorliegenden Erfindung wird auch ein Verfahren zur Herstellung von Verbindungen der Formel la' 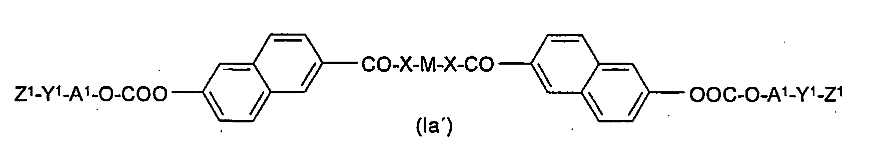 oder der Formel Ib' 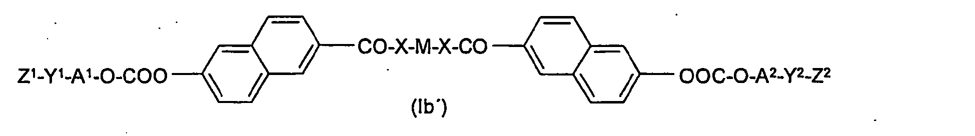 beansprucht, worin M einer Gruppierung der Formel

T²-(Y³-T²-)ₛ..

entspricht, s" Werte von 0 oder 1 annimmt, wobei für s" > 0 die Variablen T² untereinander gleich oder voneinander verschieden sein können, X und X' unabhängig voneinander Sauerstoff oder Schwefel bedeuten und T² und Y³ sowie die übrigen Variablen die allgemeine und bevorzugte Bedeutung wie zuvor definiert besitzen, welches dadurch gekennzeichnet ist, dass man eine Verbindung der Formel II'

HX-M-X'H II'

mit der entsprechenden Molzahl eines Carbonsäurederivats der Formel IIIa' 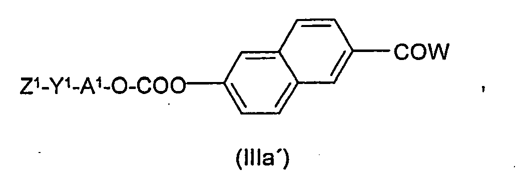 gegebenenfalls in Gegenwart von einer oder mehreren Hilfsverbindungen, zur symmetrischen Verbindung der Formel Ia' umsetzt,
oder dass man eine Verbindung der Formel II' in einem ersten Schritt mit der entsprechenden Molzahl eines Carbonsäurederivats der Formel IIIa', gegebenenfalls in Gegenwart von einer oder mehreren Hilfsverbindungen, und in einem zweiten Schritt mit der entsprechenden Molzahl eines Carbonsäurederivats der Formel IIIb' 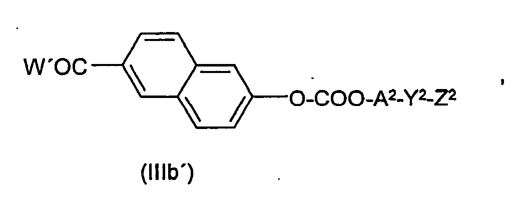 gegebenenfalls in Gegenwart von einer oder mehreren Hilfsverbindungen, zur unsymmetrischen Verbindung der Formel Ib' umsetzt, wobei W und W' für gleiche oder voneinander verschiedene, bei der Umsetzung austretende Abgangsgruppen oder für gleiche oder voneinander verschiedene Vorläufergruppen stehen, welche mit der oder den gegebenenfalls vorliegenden Hilfsverbindungen in eine austretende Abgangsgruppe überführt werden können.

Im Hinblick auf verschiedene Methoden der Estersynthese und die entsprechenden Synthesebedingungen, welche für das erfindungsgemäße Verfahren in Betracht kommen, sei auf die einschlägige und dem Fachmann bekannte Literatur, wie etwa Ullmann's Encyclopedia of Industrial Chemistry, John Wiley and Sons oder Advanced Organic Chemistry, M. B. Smith and J. March, 5th Edition, Wiley-Interscience, New York, S. 485 - 499 verwiesen.

Für W und W' kommen alle dem Fachmann als solche bekannte Gruppen in Frage.

Insbesondere sind hier für W und W' zu nennen Hydroxy, Halogen, wie etwa Fluor, Brom und insbesondere Chlor, sowie C₁-C₄-Alkoxy, wie etwa Methoxy, Ethoxy, n-Propoxy, Isopropoxy, n-Butoxy, sec-Butoxy, Isobutoxy oder tert-Butoxy.

Desweiteren kommen für W und W' Reste anorganischer Säuren, wie Hydrogensulfat, Sulfat, Dihydrogenphosphat, Hydrogenphosphat oder Phosphat, Reste organischer Säuren, wie Formiat, Acetat, Trifluoracetat, sowie Reste aliphatischer oder aromatischer Sulfonsäuren oder teilweise oder vollständig fluorierter aliphatischer oder aromatischer Sulfonsäuren in Frage. Beispiele für solche aliphatische oder aromatische Sulfonsäurereste sind etwa H₃C-SO₂-O, F₃C-SO₂-O, F₃C-CH₂-SO₂-O, F₉C₄-SO₂-O,

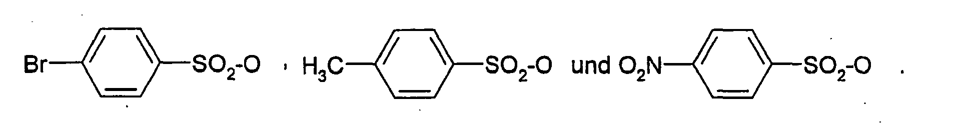

Bei den Verbindungen der Formeln IIIa' und IIIb' handelt es sich dann um die entsprechenden gemischten Anhydride.

Zur Synthese der Verbindungen der Formel Ia' kann auch von Verbindungen der Formel IIIa' ausgegangen werden, in welchen W einer Gruppe 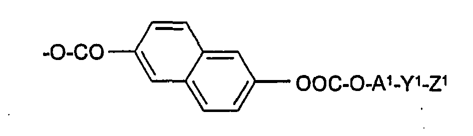 entspricht. Bei der Verbindung der Formel IIIa' handelt es sich dann um das entsprechende Säureanhydrid der Formel

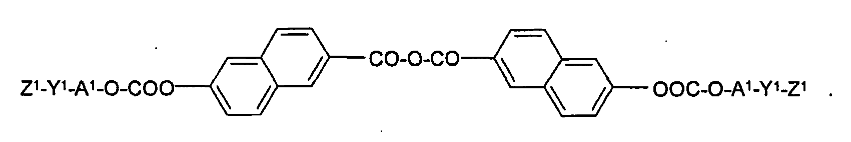

In analoger Weise kann zur Synthese der Verbindungen der Formel Ib' auch von Verbindungen der Formel IIIb' ausgegangen werden, in weichen W' einer Gruppe 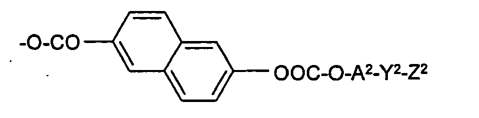 entspricht. Bei der Verbindung der Formel IIIb' handelt es sich dann ganz analog um das entsprechende Säureanhydrid der Formel

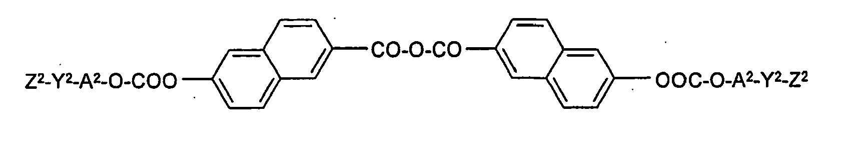

Als Hilfsverbindungen können etwa verdünnte oder konzentrierte anorganische Säuren, wie beispielsweise Schwefelsäure, Phosphorsäure oder Salzsäure, verdünnte oder konzentrierte organische Säuren, wie beispielsweise Ameisensäure, Essigsäure, Trifluoressigsäure, verdünnte oder konzentrierte aliphatische oder aromatische Sulfonsäuren, verdünnte oder konzentrierte, teilweise fluorierte aliphatische oder aromatische Sulfonsäuren oder verdünnte oder konzentrierte, vollständig fluorierte aliphatische oder aromatische Sulfonsäuren eingesetzt werden.

Als solche aliphatische oder aromatische Sulfonsäuren sind insbesondere zu nennen H₃C-SO₃H, F₃C-SO₃H, F₃C-CH₂-SO₃H, F₉C₄-SO₃H,

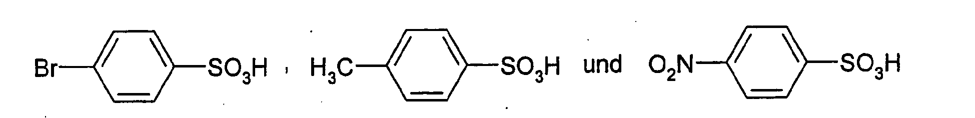

Die zuvor aufgeführten Säuren finden als Hilfsverbindungen insbesondere dann Verwendung, wenn es sich bei W und W' um Hydroxy oder C₁-C₄-Alkoxy handelt.

Für den Fall, dass es sich bei W und W' um Hydroxy handelt, sind als weitere Hilfsverbindungen zu nennen Anhydride organischer Säuren, wie beispielsweise Essigsäureanhydrid oder Trifluoressigsäureanhydrid, und Carbodiimide der Formel 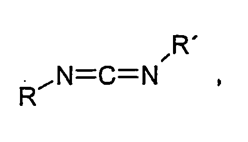 wobei die Variablen R und R' unabhängig voneinander Cyclopentyl, Cyclohexyl oder gegebenenfalls mit Hydroxy, C₁-C₄-Alkoxy, Amino, C₁-C₄-Alkylamino oder Di(C₁-C₄-Alkyl)amino substituiertes C₁-C₄-Alkyl bedeuten. Beispiele für solche Carbodiimide sind 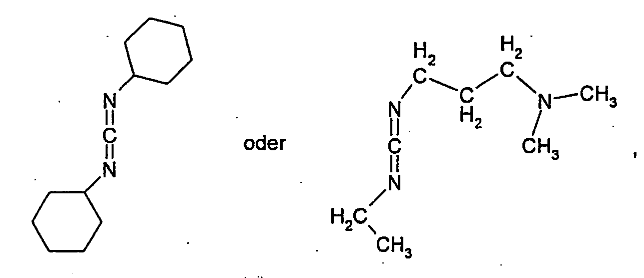 wobei die letzere Verbindung auch in Form ihres Hydrochlorids verwendet werden kann. Oftmals werden die Carbodiimide zusammen mit Aminen, wie beispielsweise N,N-Dimethylaminopyridin, verwendet. So ist ein häufig verwendetes Hilfsmittel in der Estersynthese das erstgezeigte Dicyclohexylcarbodiimid alleine oder in Kombination mit N,N-Dimethylaminopyridin. Durch die zuvor gezeigten Carbodiimide werden die Hydroxygruppen der Verbindungen der Formeln IIIa' und IIIb' beispielsweise in Gruppen der Formeln 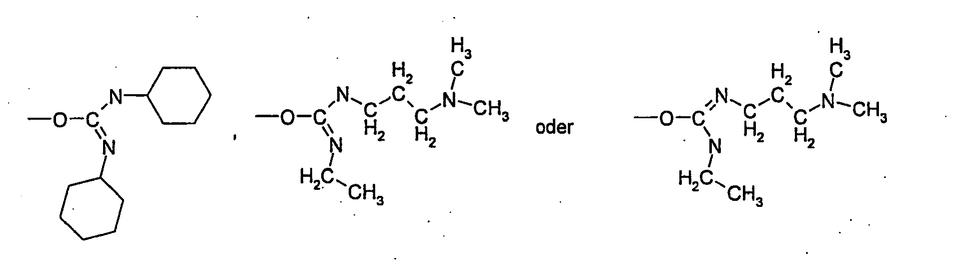 überführt. Die entsprechenden intermediären Verbindungen werden meist nicht isoliert oder sie können oft auch nicht isoliert werden, da sie schnell mit den anderen Reaktanden in der Reaktionsmischung abreagieren.

Für den Fall, dass es sich bei W und W' um Hydroxy handelt, sind weitere Hilfsverbindungen auch Dialkylazodicarboxylate in Kombination mit Triarylphosphin, wie beispielsweise Diethylazodicarboxylat/Triphenylphosphin, N,N'-Carbonyldiimidazol, aliphatische oder aromatische Sulfonsäurechloride in Kombination mit einem tertiären Amin, wie beispielsweise Methansulfonsäurechlorid/Triethylamin, Chlorsilane, Tetraphosphordecaoxid oder auch lonenaustauscher, wie beispielsweise Amberlyst^{®}-15 (kommerziell erhältlich von der Firma Rohm & Haas).

Werden als Verbindungen der Formeln IIIa' und IIIb' die zuvor angesprochenen gemischten Anhydride verwendet, so entstehen bei der Umsetzung mit Verbindung II' die entsprechenden zuvor als Hilfsverbindungen erwähnten Säuren.

Als weitere Hilfsverbindungen kommen, insbesondere wenn es sich bei W und W' um Halogen, vorzugsweise Chlor oder Brom, handelt, auch Stickstoff enthaltende fünfoder sechsgliedrige Heteroaromaten, wie etwa Imidazol oder Pyridin, in Frage. Durch diese Hereroaromaten werden die Gruppen COW bzw. COW' der Verbindungen der Formeln IIIa' und IIIb' beispielsweise in die Gruppen 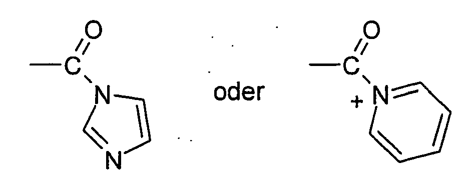 überführt. Die entsprechenden intermediären Verbindungen werden meist ebenfalls nicht isoliert bzw. sie können oft auch nicht isoliert werden, da sie schnell mit den anderen Reaktanden in der Reaktionsmischung abreagieren.

Weiterer Gegenstand der vorliegenden Erfindung sind Verbindungen der Formel 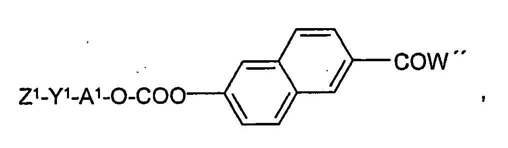 worin W" für Hydroxy, Halogen, wie beispielsweise Fluor oder Chlor, C₁-C₄-Alkoxy, wie Methoxy, Ethoxy, Propoxy, Isopropoxy, Butoxy, Isobutoxy, sec-Butoxy oder tert-Butoxy, teilweise oder vollständig halogeniertes Phenoxy, wie beispielsweise 2,4,6-Trichlorphenoxy 2,4,6-Trifluorphenoxy, Pentachlorphenoxy oder Pentafluorphenoxy, einen Rest einer anorganischen Säure, wie beispielsweise Hydrogensulfat, Sulfat, Dihydrogenphosphat, Hydrogenphosphat oder Phosphat, einen Rest einer Carbonsäure, wie beispielsweise Formiat, Acetat, Trifluoracetat, einen Rest einer aliphatischen oder aromatischen Sulfonsäure, wie beispielsweise H₃C-SO₂-O, 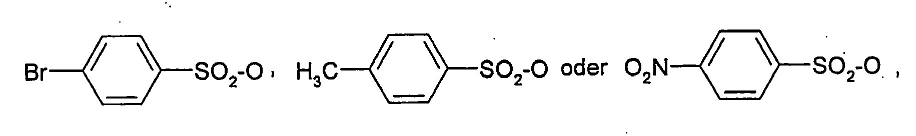 einen Rest einer teilweise oder vollständig fluorierten aliphatischen oder aromatischen Sulfonsäure, wie beispielsweise F₃C-SO₂-O, F₃C-CH₂-SO₂-O oder F₉C₄-SO₂-O, oder eine Gruppe der Formel 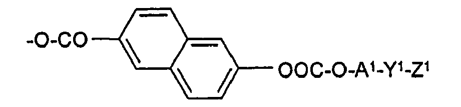 steht, und Z¹, Y¹ und A¹ dieselbe Bedeutung wie bereits weiter oben definiert besitzen.

### Beispiele:

In den nachfolgend beschriebenen Synthesen der erfingungsgemäßen Verbindungen und ihrer Edukte, sofern diese nicht kommerziell erhältlich waren, wurde Gebrauch gemacht von allgemeinen Methoden der organischen Synthese von Estern und insbesondere von mono- und difunktionellen mesogenen Verbindungen, wie sie in der einschlägigen wissenschaftlichen und Patentliteratur bekannt sind. Auch sei diesbezüglich auf die bereits zuvor zitierten Werke Ullmann's Encyclopedia of Industrial Chemistry, John Wiley and Sons und Advanced Organic Chemistry, M. B. Smith and J. March, 5th Edition, Wiley-Interscience, New York, S. 485 - 499 verwiesen.

### Synthese der erfindungsgemäßen Verbindungen:

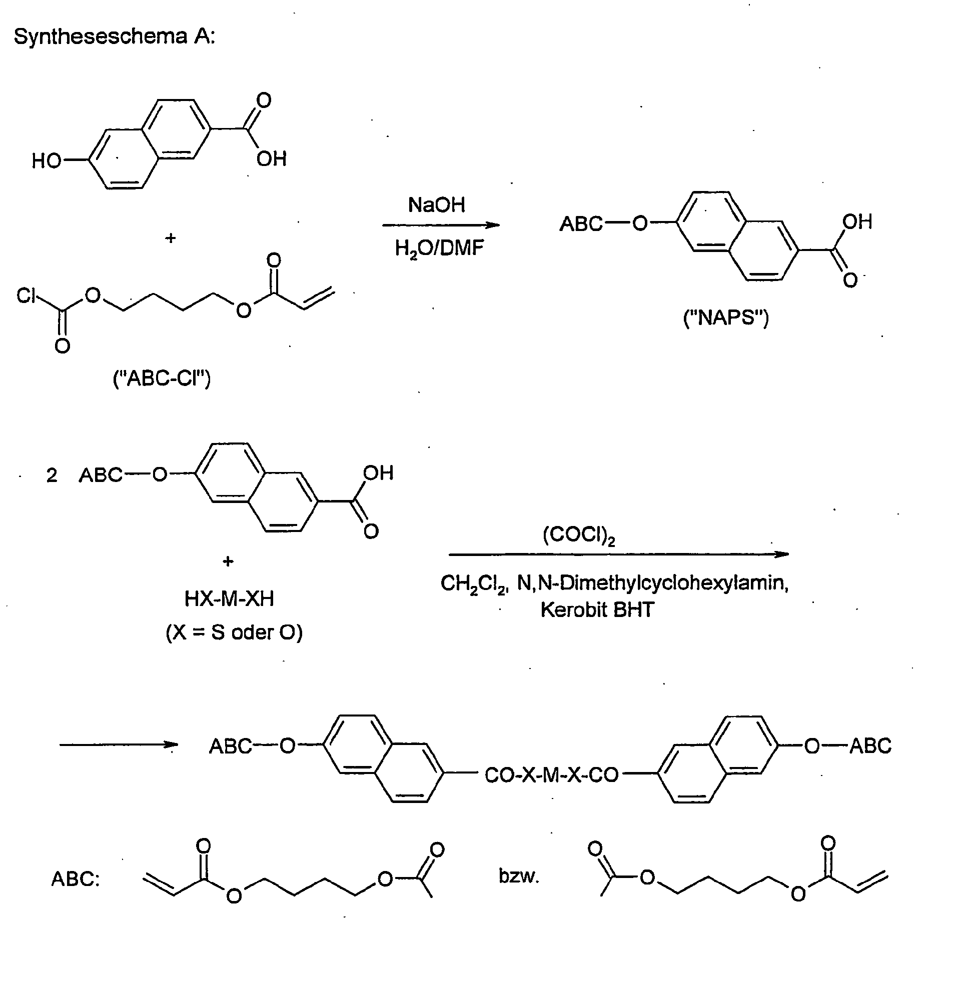

I. Herstellung von efindungsgemäßen Verbindungen der allgemeinen Formel 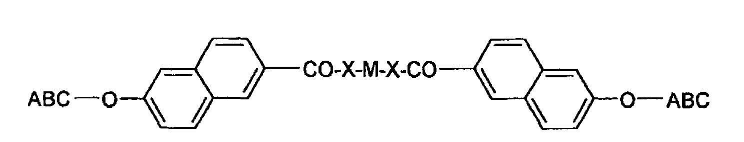 worin X-M-X die in der nachfolgenden Tabelle A1 aufgeführte Bedeutung besitzt.

Es sei hier ergänzend angemerkt, dass herstellungsbedingt sowohl in der Verbindung ABC-Cl als auch in der Gruppierung ABC der Zwischenstufe NAPS ein Teil der Acrylatgruppen in Form ihrer HCl-Addukte vorliegt. Im letzten Syntheseschritt des obigen Schemas A erfolgt jedoch wieder die Eliminierung von HCl unter Rückbildung der Acrylatgruppe (vgl. hierzu auch Beispiel 6a auf Seite 66 von WO 98/47979 A1).

In der Gruppierung ABC ("Acryloyloxy-Butyl-Chlorformiat") entspricht die Vinylgruppe des Acrylatrestes der Variablen Z¹, die Carboxylgruppe der Variablen Y¹ und der n-Butylenrest -(CH₂)₄- der Variablen A¹ gemäß der Terminologie der zuvor genannten Formel Ia'. Die Bedeutung der Variablen X und M bzw. der Gruppierung X-M-X in den synthetisierten Verbindungen ist in nachfolgender Tabelle aufgeführt.

**Tabelle A1**

| Verbindung (Edukt HX-M-XH) | X-M-X | Ausbeute (% d. Th.) |
|---|---|---|
| 1 (1E kommerziell erhältlich) | 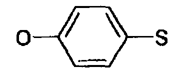 | 80 |
| 2 (2E kommerziell erhältlich) | 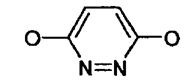 | 79 |
| 3 (3E kommerziell erhältlich) | 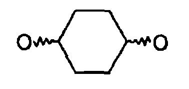 | 12 |
| 6 (6E kommerziell erhältlich) | 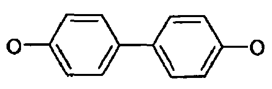 | 92 |
| 7 (7E kommerziell erhältlich) | 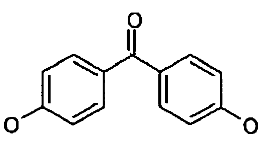 | 52 |
| 8 (8E kommerziell erhältlich) | 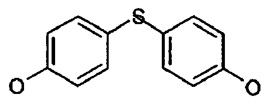 | 84 |
| 9 (9E kommerziell erhältlich) | 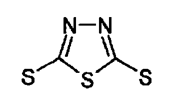 | 22 |

### Allgemeine Synthesevorschrift:

### A) Herstellung von 6-(4-Acryloyloxy-butoxycarbonyloxy)-2-naphthoesäure

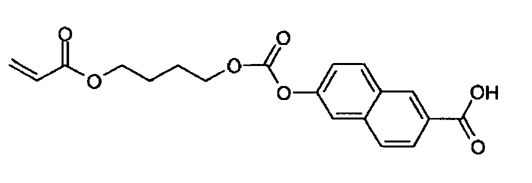

Der pH-Wert einer Lösung von 100 g (0,53 mol) 6-Hydroxy-2-naphthoesäure in einem Lösungsmittelgemisch aus 600 ml Wasser und 500 ml DMF wurde mit 10-prozentiger Natronlauge bei 0 °C auf 10 eingestellt. 132 g (0,61 mol) 4-Acryloyloxybutylchlorformiat (hergestellt gemäß WO 97/00600 A2) wurden anschließend tropfen-weise über einen Zeitraum von 2 h zugegeben, wobei man die Temperatur zwischen 0 und 5 °C und den pH-Wert mittels 10-prozentiger Natronlauge auf 10 (± 0,2 pH-Einheiten) hielt. Die resultierende Lösung wurde anschließend mit 3 I Wasser/Eis-Mischung verdünnt und der pH-Wert mit konzentrierter Salzsäure auf 5 eingestellt. Man filtrierte die erhaltene Suspension, wusch den erhaltenen Filterkuchen mit 3 I Wasser und erhielt, nach dem Trocknen, 165,8 g (87 % d.Th. basierend auf dem reinen Acrylat-Derivat) eines beige gefärbten Pulvers, welches die oben gezeigte Verbindung NAPS und das entsprechende Acrylat/HCl-Addukt enthält.

### B) Herstellung der Verbindungen der Formel

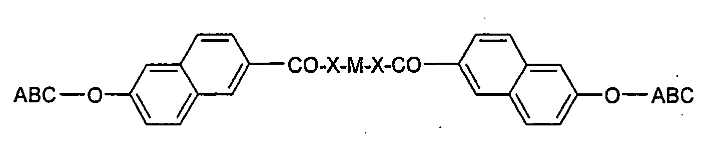

65,2 g (168 mmol) bzw. im Falle der Verbindung 10 32,6 g (84 mmol) NAPS und eine geringe Menge Kerobit^{®} BHT (0,14 g) wurden in 350 ml Oxalylchlorid vorgelegt und langsam mit 5 Tropfen Dimethylformamid (DMF) versetzt. Die Reaktionsmischung wurde anschließend eine Stunde bei Raumtempe-ratur gerührt, das überschüssige Oxalylchlorid dann im Vakuum entfernt und der Rückstand im Hochvakuum getrocknet. Das gelbliche gummiähnliche Produkt wurde in 100 ml CH₂Cl₂ gelöst, bei -10 °C unter Stickstoffspülung trop-fenweise einer Lösung aus 70 mmol der jeweiligen Verbindung HX-M-XH (Tabelle A1; Edukte 1 E bis 3E und 6E bis 9E) bzw. 70 mmol der Verbindung HX-M-Z² (Tabelle A1; Edukt 10 E), 0,03 g Kerobit^{®} BHT und 53,4 g (420 mmol) N,N-Dimethylcyclohexylamin in 200 ml CH₂Cl₂ zugegeben und die erhaltene Reaktionsmischung anschließend zuerst 12 h bei Raumtemperatur und dann 5 h bei 45 °C gerührt. Danach wurden 200 ml destilliertes Wasser und 15,2 g konzentrierte wäßrige Salzsäure zugegeben und die Phasen getrennt. Die organische Phase wurde zweimal mit je 200 ml gesättigter wässriger Kochsalzlösung gewaschen, über wasserfreiem Natriumsulfat getrocknet und im Vakuum aufkonzentriert. Der erhaltene Rückstand (in der Regel ein braunes Öl) ließ sich in der Regel durch Vermahlen oder Suspendieren in Methanol oder Ethanol, säulenchromatographisch (über SiO₂, üblicherweise als Laufmittel: Toluol/Aceton 10:1), durch Fällen aus einer Lösung in CH₂Cl₂ mittels n-Hexan oder Methanol oder einer Kombination dieser Schritte reinigen. Man erhielt das jeweilige Produkt als weißes bis grauweißes Pulver.

Ausbeute und Reinigung der jeweiligen Verbindung sind in vorstehender Tabelle A1 aufgeführt.

### II. Herstellung von erfindungsgemäßen Verbindungen der allgemeinen Formel

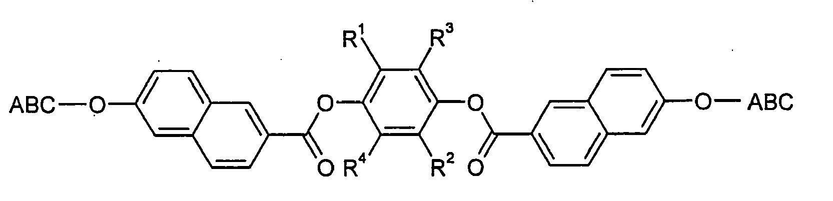

ABC entspricht, wie bereits zuvor, der Acryloyloxybutoxycarbonyl-Gruppierung, die Variablen R¹, R², R³ und R⁴ besitzen die in Tabelle A2 aufgeführten Bedeutungen.

**Tabelle A2**

| Verbindung | R¹ | R² | R³ | R⁴ | Ausbeute (%d.Th.) |
|---|---|---|---|---|---|
| 14 | Et | H | H | H | 85 |
| 15 | n-Propyl | H | H | H | 84 |
| 16 | n-Butyl | H | H | H | 55 |
| 17 | n-Hexyl | H | H | H | 65 |
| 18 | n-Octyl | H | H | H | 12 |
| 20 | Methyl | Methyl | Methyl | H | 68 |
| 21 | Methyl | Methyl | Methyl | Methyl | 18 |
| 22 | iso-Pentyl | H | H | H | 30 |
| 23 | COOEthyl | Ethy | H | H | 18 |
| 24 | tert-Butyl | H | H | H | 26 |
| 25 | F | F | F | F | 15 |
| 26 | C(O)Ethyl | H | H | H | 61 |
| 27 | C(O)Methyl | H | H | H | 79 |
| 28 | OMethyl | H | H | H | 20 |
| 29 | Cl | Cl | H | H | 48 |
| 30 | Methyl | H | Methyl | H | 23 |
| 31 | CN | H | CN | H | 83 |
| 32 | n-Octyl | n-Octyl | H | H | 35 |
| Verbindung | R¹ | R² | R³ | R⁴ | Ausbeute (% d. Th.) |
| 33 | COOMethyl | H | H | H | 85 |
| 34 | COOEthyl | H | H | H | 97 |
| 35 | COOn-Propyl | H | H | H | 84 |
| 36 | COOn-Butyl | H | H | H | 68 |
| 37 | COOn-Hexyl | H | H | H | 84 |
| 38 | Cl | H | H | H | - |

### A) Verfügbarkeit der Edukte HX-M-XH

**Tabelle A3**

| Edukt HX-M-XH | X-M-X | Edukt HX-M-XH | X-M-X |
|---|---|---|---|
| 12E kommerziell erhältlich | 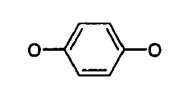 | 27E kommerziell erhältlich | 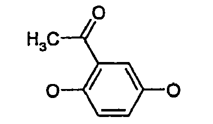 |
| 14E | 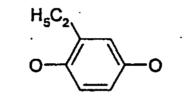 | 28E kommerziell erhältlich | 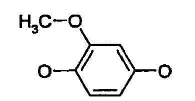 |
| 15E | 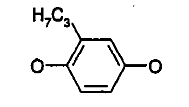 | 29E kommerziell erhältlich | 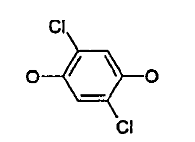 |
| 16E | 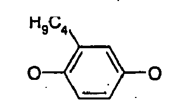 | 30E kommerziell erhältlich | 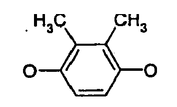 |
| 17E | 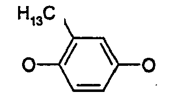 | 31E kommerziell erhältlich | 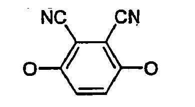 |
| 18E | 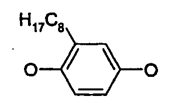 | 32E | 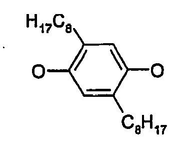 |
| 20E kommerziell erhältlich | 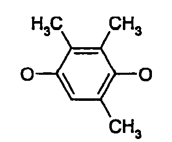 | 33E kommerziell erhältlich | 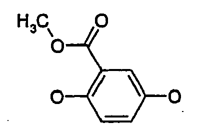 |
| 21E kommerziell erhältlich | 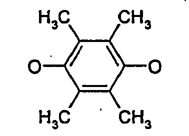 | 34E | 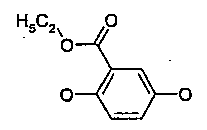 |
| 22E | 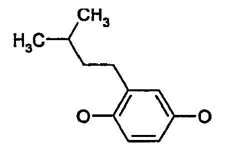 | 35E | 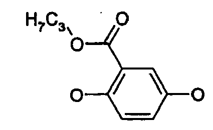 |
| 23E | 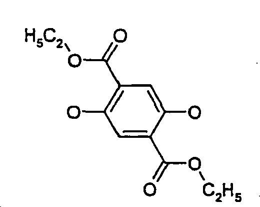 | 36E | 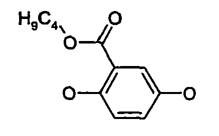 |
| 24E kommerziell erhältlich | 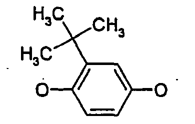 | 37E | 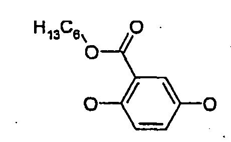 |
| 25E kommerziell erhältlich | 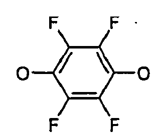 | 38E kommerziell erhältlich | 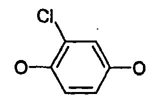 |
| 26E kommerziell erhältlich | 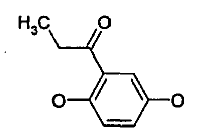 | | |

### Herstellung des Eduktes 14E

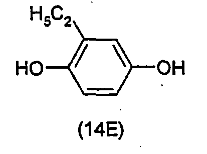

158,4 g (2,4 mol) Kaliumhydroxid wurden zu 600 ml Triethylenglykol gegeben und die resultierende Suspension auf 80 °C erhitzt. 93,1 g (0,6 mol) 2,5-Dihydroxyacetophenon und 90,1 g (1,8 mol) Hydrazin-hydrat wurden zugegeben und die resultierende Lösung für eine Dauer von 1,5 h unter Rückfluss (ca. 140 °C) gehalten. Nachdem die flüchtigen Bestandteile zwischen 140 und 190 °C abdestilliert worden waren, wurde der Ansatz 4 h bei einer Temperatur von 190 °C gehalten. Anschließend gab man 600 ml Wasser hinzu, stellte den pH-Wert mit konzentrierter Salzsäure auf 2 bis 3 und extrahierte die wässrige Phase achtmal mit je 200 ml tert-Butylmethylether. Die organischen Phasen wurden vereinigt, viermal mit je 200 ml Wasser gewaschen, über wasserfreiem Natriumsulfat getrocknet und im Vakuum aufkonzentriert. Während des Verrührens des erhaltenen braunen Öls mit n-Hexan fand Kristallisation statt. Nach der Filtration erhielt man 67,7 g (0,49 mmol, 82 % d.Th.) des gewünschten Produktes 14E als leicht bräunlichen Feststoff.

### Herstellung des Eduktes 15E

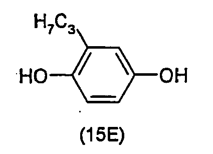

16,0 g (250,9 mmol) Kaliumhydroxid wurden zu 350 ml Triethylenglykol gegeben und die resultierende Suspension auf 80 °C erhitzt. Anschließend gab man 10 g (60,2 mmol) 2,5-Dihydroxypropiophenon und 10 g (199,2 mmol) Hydrazin-hydrat hinzu und erhitzte die Lösung 2 h unter Rückfluß (ca. 140 °C). Nachdem die flüchtigen Bestandteile zwischen 140 und 195 °C abdestilliert worden waren, wurde der Ansatz 4 h bei 195 °C gehalten. Anschließend gab man 400 ml Wasser hinzu, stellte den pH-Wert mit konzentrierter Salzsäure auf ca. 2 bis 3 und extrahierte die wässrige Phase dreimal mit je 200 ml tert-Butylmethylether. Die organischen Phasen wurden vereinigt, über wasserfreiem Magnesiumsulfat getrocknet und im Vakuum aufkonzentriert. Das erhaltene Öl wurde in CH₂Cl₂ gelöst und man gab langsam n-Hexan zu, um das Produkt auszufällen. Nach dem Filtrieren erhielt man 6,4 g (41,2 mmol, 69 % d.Th.) des gewünschten Produktes 15E als grauen Feststoff.

### Herstellung der Edukte 16E bis 18E und 22E

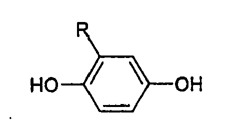

**Tabelle A4**

| Edukt HX-M-XH | R | Ausbeute (in % d.Th.) |
|---|---|---|
| 16E | n-Butyl | 50 |
| 17E | n-Hexyl | 22 |
| 18E | n-Octyl | 45 |
| 22E | 3-Methylpentyl | 54 |

### Allgemeine Synthesevorschrift:

Eine Mischung von 0,05 mol Cyclohexan-1,4-dion, 0,05 mol Aldehyd RCHO und 0,05 mol Lithiumchlorid in 100 ml Pyridin wurde 4 h auf 115 °C erhitzt. Der größte Teil des Pyridins wurde an-schließend abdestilliert, der Rückstand in 200 ml Wasser gegossen .und der pH-Wert mit konzentrierte Salzsäure auf 2 eingestellt. Die resultierende Lösung wurde zweimal mit je 200 ml Diethylether extrahiert, die vereinigten organischen Phasen über wasser-freiem Natriumsulfat getrocknet und anschließend unter reduziertem Druck aufkonzen-triert. Der braune ölige Rückstand wurde über Nacht mit n-Hexan verrührt und der resultierende Feststoff abfiltriert. Man erhielt die entsprechenden Rohprodukte als bräunliche Pulver und verwendete diese ohne weitere Reinigung für die nachfolgenden Reaktionen.

### Herstellung des Eduktes 32E

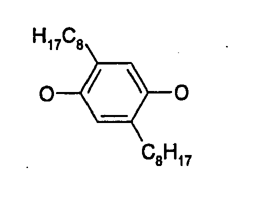

Die Herstellung erfolgte gemäß Z. Bao et al., J. Amer. Chem. Soc. 1995, 117, 12426 - 12435.

### Herstellung der Edukte 34E bis 37E

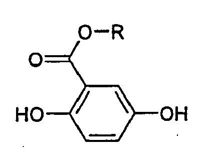

**Tabelle A5**

| Edukt HX-M-XH | R | Ausbeute (in % d.Th.) | Synthesevorschrift |
|---|---|---|---|
| 34E | Ethyl | 93 | A |
| 35E | n-Propyl | 65 | A |
| 36E | n-Butyl | 74 | B |
| 37E | n-Hexyl | 82 | B |

### Synthesevorschrift A:

Eine Mischung von 15 g (97,3 mmol) 2,5-Dihydroxybenzoesäure und 4 ml konzentrierte Schwefelsäure in 300 ml Alkohol (Ethanol für 34E und n-Propanol für 35E) wurde unter Rückfluss erhitzt. Nach 24stündiger Reaktionszeit gab man 2 ml konzentrierte Schwefelsäure hinzu und hielt den Ansatz für weitere 24 h unter Rückfluss. Nach dem Abkühlen auf Raumtemperatur gab man 300 ml Wasser zu der Reaktionsmischung, worauf das Produkt als Öl anfiel. Nach weiterem Rühren (in der Regel nach ca. einer Stunde) kristallisierte das Produkt aus und wurde nach dem Abfiltrieren als weißes Pulver erhalten.

### Synthesevorschrift B:

Eine Mischung von 3,1 g (20 mmol) 2,5-Dihydroxybenzoesäure und 5 g (59 mmol) Natriumbicarbonat in 30 ml DMF würde 1 h bei 70°C unter Stickstoff gerührt. 20 mmol des entsprechende Bromalkans R-Br wurden zugegeben und die Suspension für weitere 7 h bei 70 °C gerührt. Die Reaktionsmischung wurde auf 100 ml Wasser gegossen und die resultierende wässrige Phase mit 60 ml einer 1:1-Mischung aus n-Hexan und Essigsäureethylester extrahiert. Man wusch die organische Phase dreimalmit je 50 ml Wasser, gab 3 g Al₂O₃ (3 g) hinzu und filtrierte. Die Mutterlauge wurde unter reduziertem Druck aufkonzentriert und man erhielt das entsprechende Produkt als weißen bis grauweißen Feststoff.

Die für die jeweiligen Verbindungen gemäß Synthesevorschrift A und B erhaltenen Ausbeuten sind in der vorhergehenden Tabelle A5 aufgeführt.

### Herstellung des Edukts 23E

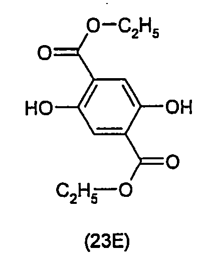

Eine Lösung von 2,0 g (10,0 mmol) 2,5-Dihydroxyterephthalsäure und 2 ml konzentrierter Schwefelsäure in 25 ml (0,42 mol) Ethanol wurde 24 h unter Rückfluss gerührt. Anschließend wurde die Reaktionsmischung auf Raumtemperatur abgekühlt und der pH-Wert mit einer 5-prozentigen wässrigen Natriumbicarbonat-Lösung auf 6 eingestellt (es wurden ca. 80 ml der Lösung benötigt), worauf ein Feststoff ausfiel. Dieser wurde abfiltriert, in Essigsäureethylester gelöst und die resultierende organische Phase zweimal mit je 100 ml Wasser und zweimal mit je 100 ml 5-prozentige wässrige Natriumbicarbonat-Lösung gewaschen, über wasserfreiem Natriumsulfat getrocknet und im Vakuum aufkonzentriert. Man erhielt einen bräunlichen Rückstand, der hauptsächlich aus der Verbindung 23E bestand und ohne weitere Reinigung für die nachfolgenden Reaktionen verwendet wurde.

### B) Herstellung von erfindungsgemäßen Verbindungen der Formel

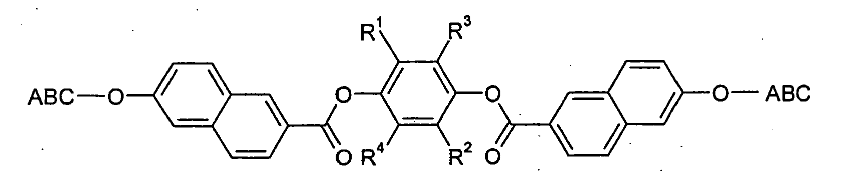

65,2 g (168 mmol) NAPS und eine geringe Menge Kerobit^{®} BHT (0,14 g) wurden langsam in 350 ml Oxalylchlorid, welches mit 5 Tropfen Dimethylformamid (DMF) versetzt worden war, eingebracht. Die Reaktionsmischung wurde anschließend eine Stunde bei Raumtemperatur gerührt, das überschüssige Oxalylchlorid dann im Vakuum entfernt und der Rückstand im Hochvakuum getrocknet. Das gelbliche gummiähnliche Produkt wurde in 100 ml CH₂Cl₂ gelöst, bei -10°C unter Stickstoffspülung tropfenweise einer Lösung aus 70 mmol der jeweiligen Verbindung HX-M-XH (zur Verfügbarkeit dieser Verbindungen siehe Tabelle A3), 0,03 g Kerobit^{®} BHT und 53,4 g (420 mmol) N,N-Dimethylcyclohexylamin in 200 ml CH₂Cl₂ zugegeben und die erhaltene Reaktionsmischung anschließend zuerst 12 h bei Raumtemperatur und dann 5 h bei 45°C gerührt. Danach wurden 200 ml destilliertes Wasser und 15,2 g konzentrierte wäßrige Salzsäure zugegeben und die Phasen getrennt. Die organische Phase wurde zweimal mit je 200 ml gesättigter wässriger Kochsalzlösung gewaschen, über wasserfreiem Natriumsulfat getrocknet und im Vakuum aufkonzentriert. Der erhaltene Rückstand (in der Regel ein braunes Öl) ließ sich in der Regel durch Vermahlen oder Sus-pendieren in Methanol oder Ethanol, säulenchromatographisch (über SiO₂, üblicherweise als Laufmittel: Toluol/Aceton 10:1), durch Fällen aus einer Lösung in CH₂Cl₂ mittels n-Hexan oder Methanol oder einer Kombination dieser Schritte reinigen. Man erhielt das jeweilige Produkt als weißes bis grauweißes Pulver.

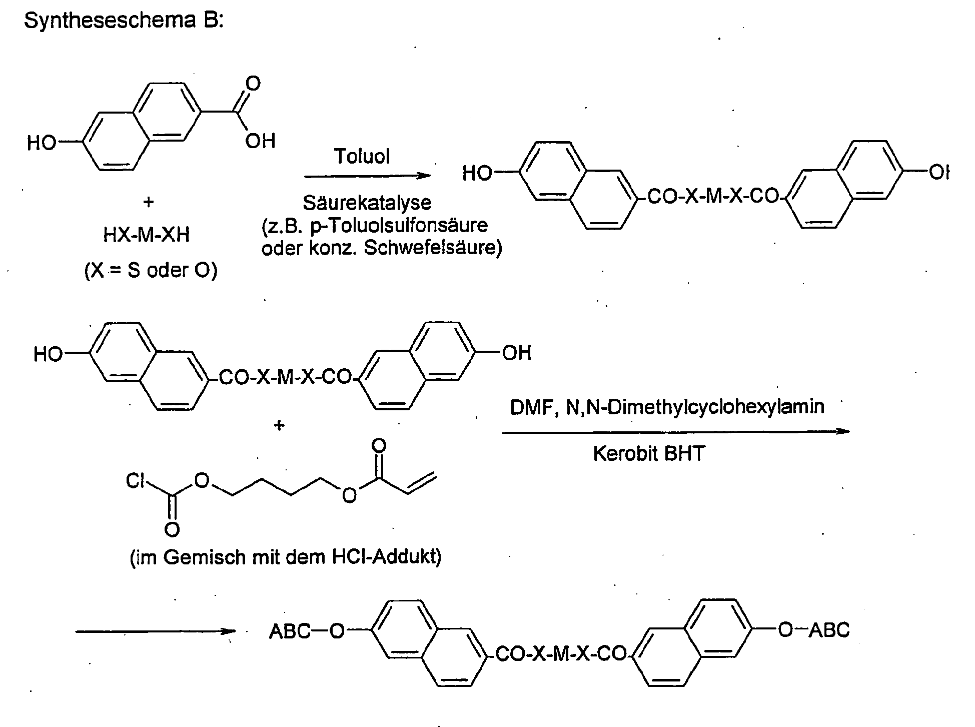

### III. Herstellung von erfindungsgemäßen Verbindungen der allgemeinen Formel

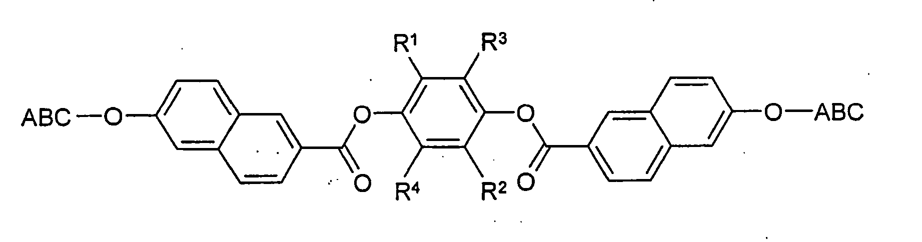

wobei die Gruppierung 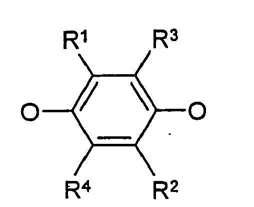 der Einheit X-M-X in Schema B entspricht und die Variablen R¹ bis R⁴ die in Tabelle B1 aufgeführte Bedeutung besitzen.

**Tabelle B1**

| Verbindung | R¹ | R² | R³ | R⁴ |
|---|---|---|---|---|
| 13 | Methyl | H | H | H |
| 19 | Methyl | Methyl | H | H |
| 21 | Methyl | Methyl | Methyl | Methyl |
| 38 | Cl | H | H | H |

### A) Verfügbarkeit der Hydrochinone der Formel

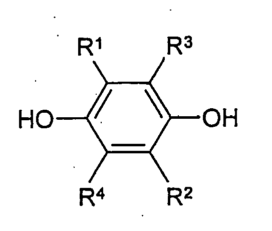

**Tabelle B2**

| Edukt HX-M-XH | R¹ | R² | R³ | R⁴ |
|---|---|---|---|---|
| 13E kommerziell erhältlich | Methyl | H | H | H |
| 19E | Methyl | Methyl | H | H |
| 21E kommerziell erhältlich | Methyl | Methyl | Methyl | Methyl |
| 38E kommerziell erhältlich | Cl | H | H | H |

### Herstellung des Eduktes 19E

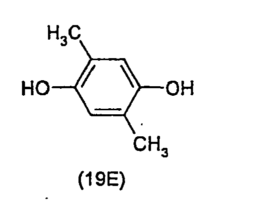

Eine Suspension von 75,7 g (0,435 mol) Natriumdithionit in 150 ml Wasser wurde langsam einer Lösung von 5,0 g 2,5-Dimethyl-p-benzochinon in 150 ml Diethylether zugegeben. Die Reaktionsmischung wurde eine Stunde lang kräftig gerührt, wobei sich die Farbe von gelb nach farblos veränderte. Anschließend wurden 200 ml Wasser zugegeben bis der gesamte Feststoff gelöst war und für eine weitere Stunde gerührt. Die Phasen wurden separiert und die wässrige Phase zweimal mit je 100 ml Diethylether extrahiert. Die vereinigten organischen Phasen wurden mit 100 ml Wasser und 100 ml konzentrierter Kochsalzlösung gewaschen, über wasserfreiem Magnesiumsulfat getrocknet und unter reduziertem Druck aufkonzentriert. Der feste Rückstand wurde in 100 ml CH₂Cl₂ suspendiert, die Suspension eine Stunde gerührt und dann der Feststoff abfiltriert. Man erhielt in nahezu quantitativer Ausbeute 5,1 g der gewünschten Verbindung 19E als weißes Pulver.

### B) Herstellung der Dihydroxyverbindungen der Formel

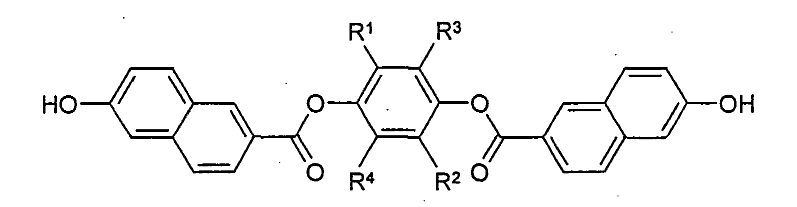

**Tabelle B3**

| R¹ | R² | R³ | R⁴ | Ausbeute (% d. Th.) |
|---|---|---|---|---|
| Methyl | H | H | H | 14 |
| Methyl | Methyl | H | H | 53 |
| Methyl | Methyl | Methyl | Methyl | 84 |
| Cl | H | H | H | 90 |

Eine Mischung von 0,06 mol (11,3 g) 6-Hydroxy-2-naphthoesäure, 0,033 mol des entsprechenden Hydroquinonderivats 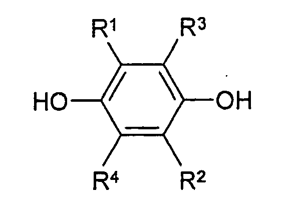 und 4 Tropfen konzentrierter Schwefelsäure in 40 ml Toluol wurde unter azeotropen Destillationsbedingungen (Dean-Stark) für 3 h unter Rückfluss erhitzt. Nach dem Abkühlen auf Raumtemperatur gab man 100 ml Methanol hinzu und filtrierte die resultierende Lösung. Der Filterkuchen wurde mehrmals mit Methanol gewaschen und anschließend getrocknet. Man erhielt die entsprechende Verbindung als leicht bräunlich gefärbten Feststoff.

### C) Herstellung der erfindungsgemäßen Verbindungen der Formel

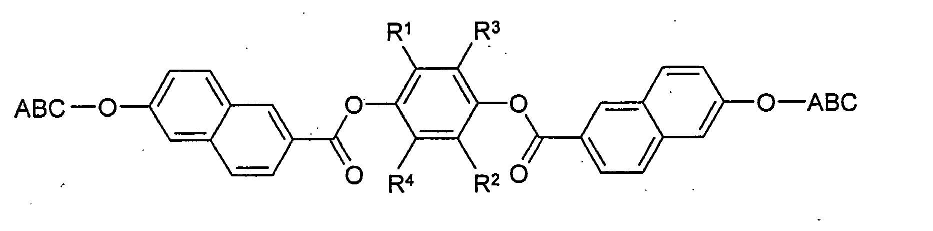

**Tabelle B4**

| Verbindung | Ausbeute (% d. Th.) |
|---|---|
| 13 | 39 |
| 19 | 85 |
| 21 | 67 |
| 38 | 72 |

22,1 mmol 4-Acryloyloxybutylchlorformiat wurden bei 0 °C tropfenweise zu einer Lösung von 6,3 mmol der entsprechenden unter B) hergestellten Dihydroxyverbindung und 18,9 mmol N,N-Dimethylcyclohexylamin in 20 ml DMF zugegeben und die Mischung 12 h bei Raumtemperatur gerührt. Dann wurden nochmals 6,3 mmol N,N-Dimethylcyclohexylamin zugegeben und der Reaktionsansatz eine weitere Stunde bei 40 °C gerührt. Die Reaktionsmischung wurde auf 300 ml Wasser gegossen und der pH-Wert mit konzentrierter Salzsäure auf 5 eingestellt. Nach zweistündigem Rühren wurde die Suspension filtriert, der Filterkuchen mit Wasser gewa-schen und getrocknet. Nach der säulenchromatographischen Reinigung (SiO₂, Toluol/ Essigsäureethylester 20: 1) erhielt man die entsprechenden Verbindungen als weiße Feststoffe in den in der Tabelle angegebenen Ausbeuten.

### IV. Herstellung der erfindungsgemäßen Verbindung 11 der Formel

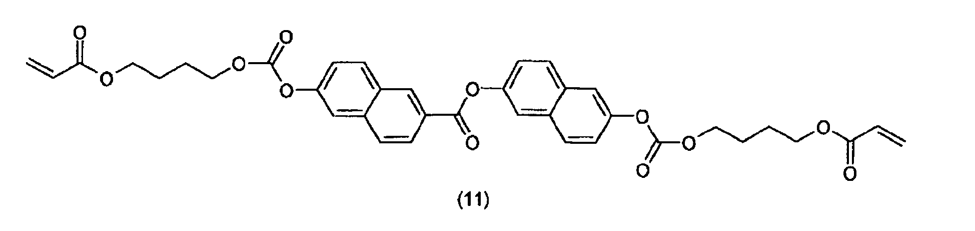

Im Vergleich zwischen der allgemeinen Formel I und der Formel von Verbindung 11 entsprechen die Vinylgruppen der Acrylatreste den Variablen Z¹ und Z² , die Carboxylgruppen der Acrylatreste den Variablen Y¹ und Y², die n-Butylenreste -(CH₂)₄- den Variablen A¹ und A² und die rechte Carbonatgruppe entspricht der Variablen Y⁵. Die Variable r in Formel I nimmt den Wert 0 an und den Variablen Y³ und T² in der Gruppierung (Y³-T²-)ₛ kommt die Bedeutungen einer-CO-O- bzw. 2,6-Naphthylen-Gruppe zu, wobei die Variable s einen Wert von 1 annimmt.

### A) Herstellung der Verbindung

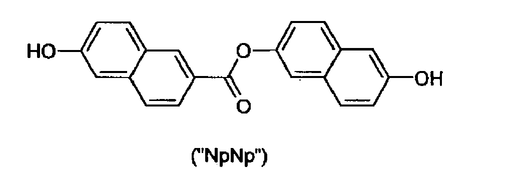

Eine Mischung von 9,41 g (0,05 mol) 6-Hydroxy-2-naphthoesäure, 24,03 g (0,15 mol) 2,6-Dihydroxynaphthalin, 0,8 g p-Toluolsulfonsäure-monohydrat und 3 Tropfen konzentrierter Schwefelsäure in 100 ml Toluol wurde 12 h bei gelegentlicher Zugabe von konzentrierter Schwefelsäure (3 Tropfen alle 3 h) unter Rückfluss erhitzt. Die erhaltene Suspension wurde auf Raumtemperatur abgekühlt und filtriert. Der Filterkuchen wurde in Methanol suspendiert, die Suspension 3 h gerührt und schließlich der Feststoff abfiltriert. Man erhielt 10,9 g 6-Hydroxy-2-naphthoesäure-6-hydroxynaphthylester ("NpNp") als bräunlichen Feststoff, welcher ohne weitere Reinigung im Folgeschritt verarbeitet wurde.

### B) Herstellung der erfindungsgemäßen Verbindung 11

10 g (0,048 mol) 4-Acryloyloxybutylchlorformiat wurden bei 0 °C tropfenweise zu einer Lösung von 6,6 g (20 mmol) NpNp, 7,6 g (0,06 mol) N,N-Dimethylcyclohexylamin und 0,03 g 4-Hydroxy-TEMPO in 40 ml DMF gegeben. Nachdem die Reaktionsmischung eine weitere Stunde bei dieser Temperatur gerührt worden war, ließ man sie langsam auf Raumtemperatur erwärmen. Nach vierstündigem Erhitzen bei 40 °C wurden 150 ml Wasser zugegeben und der pH-Wert mit konzentrierter Salzsäure auf 4 bis 5 eingestellt. Nachdem die erhaltene Mischung dekantiert und die verbleibende viskose Paste über Nacht mit 50 ml Methanol gerührt worden war, erhielt man einen braunen Feststoff, welcher säulenchromatographisch (SiO₂, Toluol/Aceton 10:1) gereinigt wurde. Man erhielt 7,7 g (62 % d.Th.) der gewünschten Verbindung 11 in Form eines weißen Feststoffes.

### Bestimmung des Phasenverhaltens der erfindungsgemäßen Verbindungen:

Das Phasenverhalten der erfindungsgemäßen Verbindungen beim Aufheizen und Abkühlen ist der nachfolgenden Tabelle C1 zu entnehmen. Die Bezeichnung der Phasen erfolgte entsprechend der allgemein verwendeten Notation (vgl. hierzu z.B. Pure Appl. Chem. 2001, 73, 845-895; Ullmann's Encyclopedia of Industrial Chemistry, Sixth Edition, 2000 Electronic Release, Chap 3.1 "Thermotropic Liquid Crystal Polymor-phism"; Angew. Chem. 2004, 116, 6340-6368). Zur Bezeichnung der kristallinen Phase wurde "K" gewählt, "poly" weist darauf hin, dass Polymerisation stattfindet bzw. stattgefunden hat. "Δn" bezeichnet die Anisotropie des Brechungsindex der jeweiligen nematischen Verbindung.

Die Δn-Werte wurden, wenn nicht anders vermerkt, bei 20°C nach gängigen Verfahren bestimmt (beispielsweise S. T. Tang, H. S. Kwok, J. App. Phys. 2003, 89, 1, 80 ff und G. Pelzl in "Handbook of Liquid Crystals", Vol 2A , Chap. 2.4 "Optical Properties of Nematic Liquid Crystals", 128-141, 1998, Eds. D. Demus, J. W. Gooby, G. W. Gray, H. W. Spiess, V. Vill, Wiley-VCH sowie in die darin zitierten Publikationen).

**Tabelle C1**

| Verbindung | Phasenverhalten | | Δn (20°C) |
|---|---|---|---|
| | Aufheizen | Bemerkung | |
| 1 | K 68 poly | - | - |
| 2 | K 128 -131 N 144 -150 poly | - | - |
| 3 | K74-120 I | - | - |
| 6 | K 150 N poly | - | - |
| 7 | poly | - | - |
| 8 | poly | - | - |
| 9 | K 134 poly | - | - |
| 11 | K 68 - 77 N 144 -150 I | Verbindung lässt sich unterkühlen | 0,19 |
| 12 | K 105 N 220 poly | - | - |
| 13 | K 103-105N poly | - | 0,20 |
| 14 | K 86 - 87 N 174 - 179 I | Verbindung lässt sich unterkühlen | 0,20 |
| 15 | K 73 - 76 N 155 -158 I | Verbindung lässt sich unterkühlen | 0,20 |
| 16 | K 72 - 76 N 143 -147 poly | Verbindung lässt sich unterkühlen | 0,19 |
| 17 | K 65 - 75 N 131 -133 poly | Verbindung lässt sich unterkühlen | 0,18 |
| 18 | K 53 - 56 N 108 -111 I | Verbindung lässt sich unterkühlen | 0,16 |
| 19 | K 118 -130 N 180 poly | Verbindung lässt sich unterkühlen | - |
| 20 | K 109 -112 N 180 poly | Verbindung lässt sich - unterkühlen | - |
| 21 | K 150 -156 N 200 poly | Verbindung lässt sich unterkühlen | - |
| 22 | K 78 - 82 N 139 -141 I | Verbindung lässt sich unterkühlen | - |
| 23 | K 140 -144 I | monotrope Mesophase | - |
| 24 | K 117 -118 N 145 -149 poly | Verbindung lässt sich unterkühlen | - |
| 25 | K 114 - 122 N poly | - | - |
| 26 | K 90 poly | - | - |
| 27 | K 98 -102 N 160 poly | Verbindung lässt sich unterkühlen | - |
| 28 | K 84 - 90 N 172 -180 I | Verbindung lässt sich unterkühlen | 0,18 |
| 29 | K 142 N poly | - | - |
| 30 | Schmelzpunkt: 93-96 °C Klarpunkt: 113°C | Verbindung lässt sich unterkühlen | - |
| 31 | K 146 -161 N poly | - | - |
| 32 | K 65 - 69 I | - | - |
| 33 | K 106 -109 N 130 poly | Verbindung lässt sich unterkühlen | 0,21 |
| 34 | K 68 - 71 N 139 poly | Verbindung lässt sich unterkühlen | 0,20 |
| K 35 | 65 - 78 N 140 poly | Verbindung lässt sich unterkühlen | 0,19 |
| 36 | K 56 - 63 N 155- 156 I | Verbindung lässt sich unterkühlen Verbindung lässt sich | 0,18 |
| 37 | K 58 - 63 N 120 poly | unterkühlen | 0,18 |
| 38 | K 105 N poly | - | - |

Das Phasenverhalten ausgewählter chiral-nematischer Zusammensetzungen beim Aufheizen und Abkühlen ist der nachfolgenden Tabelle C2 zu entnehmen. Die chiral-nematische Zusammensetzung (in der Tabelle C2 wurde die auftretende chiral-nematische Phase mit "N*" bezeichnet) wurde durch Mischen von 6 Mol-% des Dotierstoffes der Formel (39) 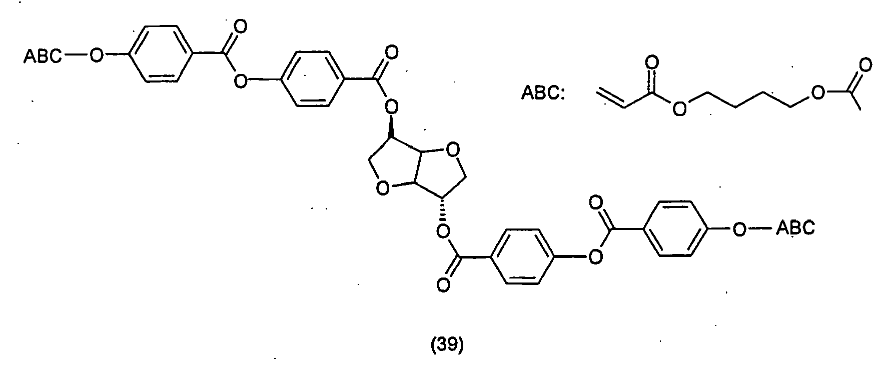 mit 94 Mol-% der entsprechenden, in Tabelle C1 charakterisierten Verbindung erhalten.

**Tabelle C2**

| Zusammen setzung | Verbindung | Dotierstoff | Phasenverhalten |
|---|---|---|---|
| 1 | 15 | 39 | K 77-80 N* 120 poly |
| 2 | 17 | 39 | <30 N* 123-127 I |
| 3 | 33 | 39 | K 96-106 N* 138 poly |
| 4 | 34 | 39 | <30 N* 131 poly |

Den Tabellen C1 und C2 kann entnommen werden, dass insbesondere Verbindungen mit lateralen Substituenten im mesogenen Molekülteil, wie etwa Alkylresten (vgl. Verbindungen 13 bis 18 in Tabelle C1 und Zusammensetzungen 1 und 2 in Tabelle C2) oder Alkoxycarbonylresten (vgl. Verbindungen 33 bis 37 in Tabelle C1 und Zusammensetzungen 3 und 4 in Tabelle C2), mit zunehmender Alkylkettenlänge des lateralen Substituenten einen breiteren Existenzbereich der nematischen bzw. chiral-nematischen Phase, tendenziell niedrigere Schmelz- und Klärpunkte sowie ausgeprägtere Unterkühlungseffekte aufweisen, was für die anwendungstechnischen Eigenschaften der erfindungsgemäßen Verbindungen und Flüssigkristallzusammensetzungen von Vorteil ist.

Darüberhinaus lassen sich mit den zuvor beschriebenen erfindungsgemäßen nematischen Verbindungen bzw. chiral-nematischen Zusammensetzungen Polymerfilme in geringen Dicken bis hinunter zu wenigen Mikrometern herstellen, welche eine ausgezeichnete homogene nematische bzw. chiral-nematische Orientierung und hohe Beständigkeit aufweisen. Diese Filme werden beispielsweise durch Aufrakeln von Lösungen dieser Verbindungen bzw. Zusammensetzungen (als Lösungsmittel kommen etwa in Frage Methylethylketon, Tetrahydrofuran oder Toluol sowie Mischungen dieser Lösungsmittel) auf Polyethylenterephthalat(PET)- oder Triacetylcellulose(TAC)-Substrate - es handelt sich hier meist um Folien- und anschließende UV-Härtung erhalten. Zur genauen Verfahrensweise hinsichtlich der Herstellung solcher Filme sei beispielsweise auf die Schrift WO 99/11733 A1 verwiesen.

## Patentansprüche

1. Verbindungen der allgemeinen Formel I 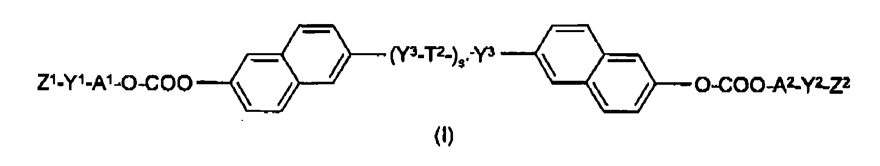 in welcher die Variablen folgende Bedeutung haben:
Z¹, Z² unabhängig voneinander Wasserstoff, gegebenenfalls substituiertes C₁-C₂₀-Alkyl, in welchem die Kohlenstoffkette durch Sauerstoffatome in Etherfunktion, Schwefelatome in Thioetherfunktion oder durch nichtbenachbarte imino- oder C₁-C₄-Alkyliminogruppen unterbro- chen sein kann, oder reaktive Reste, über die eine Polymerisation herbeigeführt werden kann,
A¹, A² unabhängig voneinander Spacer mit 1 bis 30 Kohlenstoffatomen, in welchen die Kohlenstoffkette durch Sauerstoffatome in Etherfunktion, Schwefelatome in Thioetherfunktion oder durch nichtbenachbarte Imino- oder C₁-C₄-Alkyliminogruppen unterbrochen sein kann,
Y¹ Y² unabhängig voneinander eine chemische Einfachbindung, Sauerstoff, Schwefel, -CO-, -O-CO-, -CO-O-, -S-CO-, -CO-S-, -NR-CO- oder -CO-NR-,
Y³ eine chemische Einfachbindung, Sauerstoff, Schwefel, -CR=CR-, C≡C-, -CR=CR-CO-O-, -O-CO-CR=CR-, -C≡C-O-, -O-C≡C-, -CH₂-CH₂-, -CH₂-O-, -O-CH₂-, -CH₂-S-, -S-CH₂-, -CO-, -O-CO-, -CO-O-, -S-CO-, -CO-S-, -NR-CO-, -CO-NR-, -O-CO-O-, -O-CO-NR- oder -NR-CO-O-,
R Wasserstoff oder C₁-C₄-Alkyl,
T² zweiwertige gesättigte oder ungesättigte, gegebenenfalls substituierte iso- oder heterocyclische Reste,
und
s' Werte von 0, 1 oder 2,
wobei für s' > 0 die Variablen Y³ und für s' > 1 die Variablen T² untereinander gleich oder voneinander verschieden sein können
und
sowohl die Wasserstoffatome im 2,6-Naphthylrest als auch die an Kohlenstoffatome gebundenen Wasserstoffatome in den Variablen Z¹, Z², A¹, A², Y³, R und T² teilweise oder vollständig durch Halogenatome substituiert sein können.

2. Verbindungen nach Anspruch 1, in welchen die Variable T² bedeutet 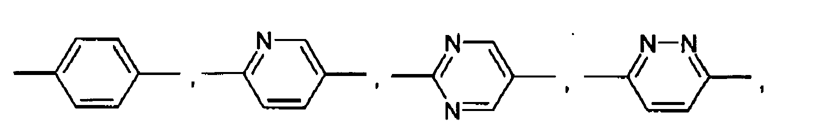 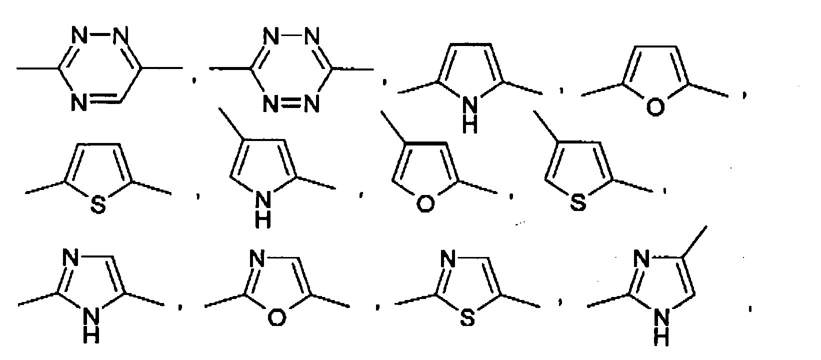 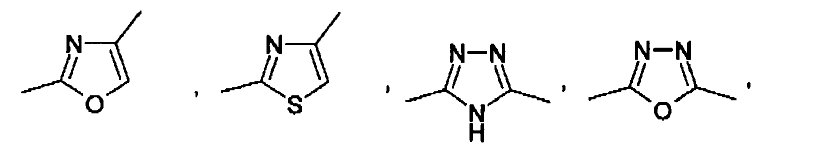 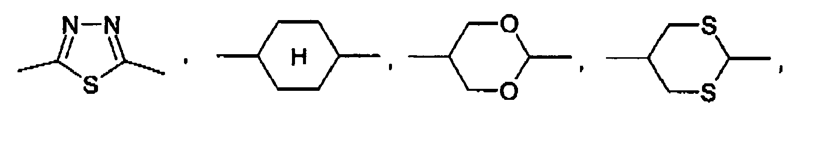 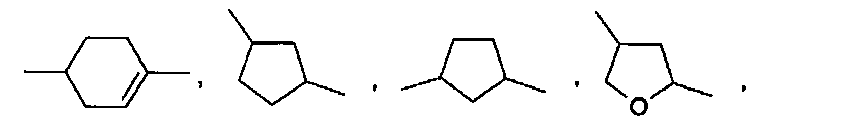 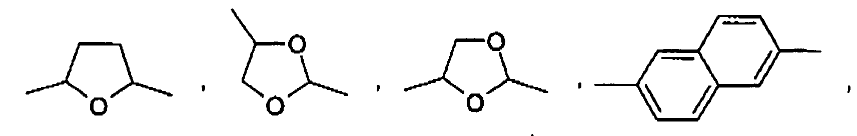 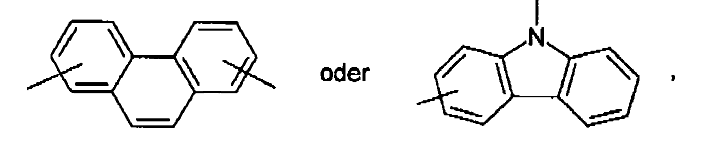 wobei die Reste 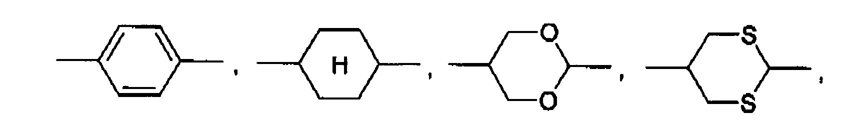 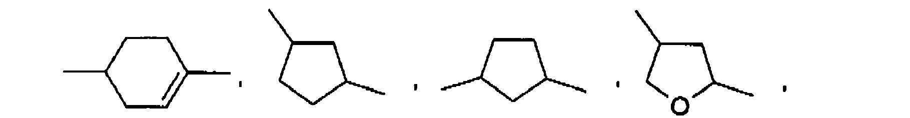 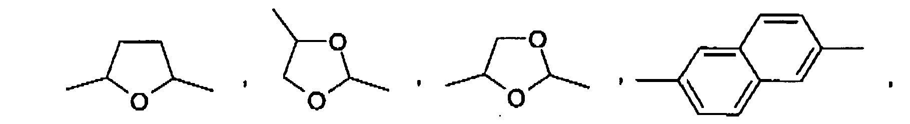 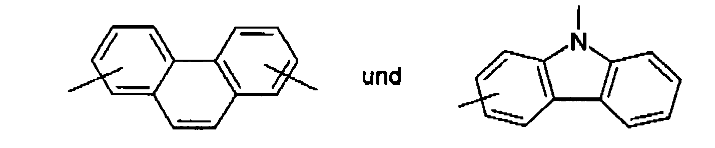 mit bis zu vier gleichen oder verschiedenen Substituenten,
der Rest 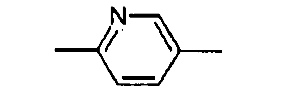 mit bis zu drei gleichen oder verschiedenen Substituenten,
die Reste 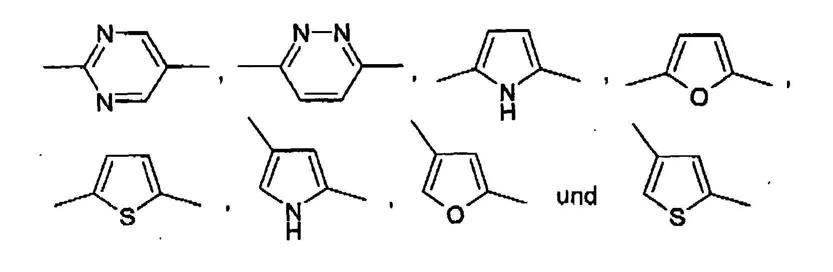 mit bis zu zwei gleichen oder verschiedenen Substituenten
und
die Reste 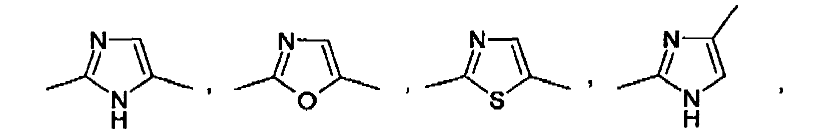 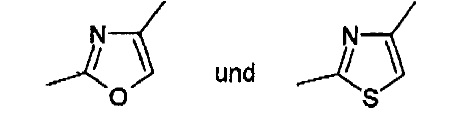 mit einem Substituenten
Halogen, NO₂, NO, CN, CHO, L¹, CO-L¹, X¹-CO-L¹, X¹-SO-L¹, X¹-SO₂-L¹, X¹-L¹, CO-X¹-L^{1'}, O-CO-X¹-L^{1'}, SO-X¹-L^{1'} oder SO₂-X¹-L^{1'} substituiert sein können, wobei bedeuten
L¹ C₁-C₂₀-Alkyl, C₂-C₂₀-Alkenyl, C₂-C₂₀-Alkinyl, C₆-C₁₀-Aryl, Heteroaryl mit 2 bis 12 Kohlenstoffatomen, C₆-C₁₀-Aryl-C₁-C₂₀-alkyl, C₆-C₁₀-Aryl- C₂-C₂₀-alkenyl, C₆-C₁₀-Aryl-C₂-C₂₀-alkinyl, Heteroaryl-C₁-C₂₀-alkyl, He- teroaryl-C₁-C₂₀-alkenyl oder Heteroaryl-C₁-C₂₀alkinyl mit jeweils 2 bis 12 Kohlenstoffatomen im Heteroarylrest, wobei die C₁-C₂₀-Kohlen- stoftkette durch Sauerstoffatome in Etherfunktion. Schwefelatome in Thioetherfunktion, nichtbenachbarte Imino-, C₁-C₂₀-Alkylimino- und/oder Carbonylgruppen unterbrochen sein kann und sowohl das C₈-C₁₀-Aryl als auch das Heteroaryl mit einem oder mehreren Substi- tuenten ausgewählt aus der Gruppe bestehend aus Halogen, NO₂, NO, CN, CHO, L², CO-L², X²CO-L², X²-SO-L², X²-SO₂-L², X²-L^{2'}, CO-X²-L^{2'}, O-CO-X²-L^{2'}, SO-X²-L^{2'} und SO₂X²-L^{2'} substituiert sein kann,
L^{1'} Wasserstoff oder unabhängig von L¹ die gleiche Bedeutung wie L¹.
L² C₁-C₂₀-Alkyl, C₂-C₂₀-Alkenyl, C₂-C₂₀-Alkinyl, C₆-C₁₀-Aryl, Heteroaryl mit 2 bis 12 Kohlenstoffatomen, C₆-C₁₀-Aryl-C₁-C₂₀-alkyl, C₆-C₁₀-Aryl- C₂-C₂₀-alkenyl, C₆₋C₁₀-Aryl-C₂-C₂₀-alkinyl, Heteroaryl-C₁-C₂₀-alkyl, He- teroaryl-C₂C₂₀-alkenyl oder Heteroaryl-C₂-C₂₀-alkinyl mit jeweils 2 bis 12 Kohlenstoffatomen im Heteroarylrest,
L^{2'} Wasserstoff oder unabhängig von L² die gleiche Bedeutung wie L²
und
X¹, X² unabhängig voneinander Sauerstoff, Schwefel oder NL^{1'} bzw. NL^{2'},
wobei in den Resten L¹ und/oder L² die an Kohlenstoffatome gebundenen Wasserstoffatome teilweise oder vollständig durch Halogenatome substituiert sein können.

3. Verbindungen nach Anspruch 1 oder 2, in welchen die Gruppierungen Z¹-Y¹-A¹- und -A²-Y²-Z² gleich sind.

4. Verbindungen nach einem oder mehreren der Ansprüche 1 bis 3, in welchen Z¹ und/oder Z² reaktive Reste 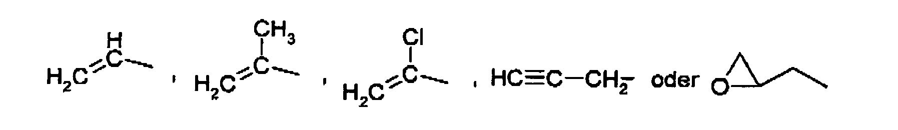 bedeuten.

5. Verbindungen nach einem oder mehreren der Ansprüche bis 3, in welchen Z¹ und/oder Z² reaktive Reste 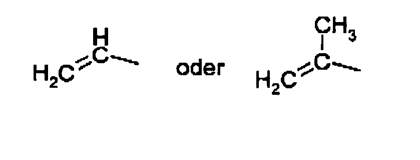 bedeuten.

6. Verbindungen nach einem oder mehreren der Ansprüche 1 bis 3, in weichen Z¹-Y¹ und/oder Z²-Y² reaktive Gruppierungen 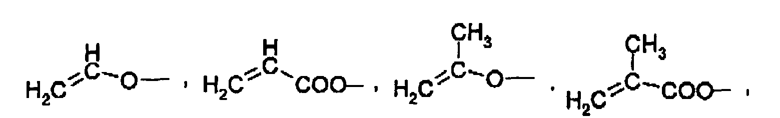 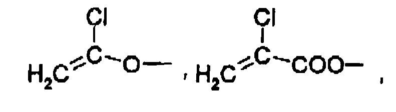 HC≡C-CH₂-O-, HC≡C-CH₂-COO-, 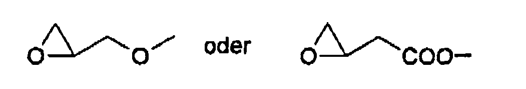 bedeuten.

7. Verbindungen nach einem oder mehreren der Ansprüche 1 bis 3, in welchen Z¹-Y¹ und/oder Z²-Y² reaktive Gruppierungen 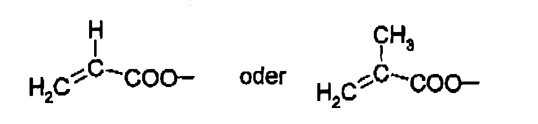 bedeuten.

8. Polymerisierbare oder nicht polymerisierbare flüssigkristalline Zusammensetzung, enthaltend als Komponenten 10 bis 100 Gew.-% der Verbindungen gemäß den Ansprüchen 1 bis 7, 0 bis 90 Gew.-% weitere Monomere, 0 bis 50 Gew-% einer oder mehrerer chiraler Verbindungen und 0 bis 90 Gew.-% weitere Zusätze, wobei die Summe der Anteile der Komponenten sich zu 100 Gew.-% ergänzt.

9. Oligomer oder Polymer, erhältlich durch Oligomerisation oder Polymerisation einer polymerisierbaren flüssigkristallinen Zusammensetzung gemäß Anspruch 8.

10. Verfahren zum Bedrucken oder Beschichten eines Substrats, **dadurch gekennzeichnet, dass** man eine polymerisierbare flüssigkristalline Zusammensetzung gemäß Anspruch 8 auf das Substrat aufbringt und anschließend polymerisiert.

11. Verwendung einer flüssigkristallinen Zusammensetzung gemäß Anspruch 8 oder eines Oligomers oder Polymers gemäß Anspruch 9 zur Herstellung optischer oder elektrooptischer Bauteile.

12. Verfahren zur Herstellung von Verbindungen der Formel Ia' 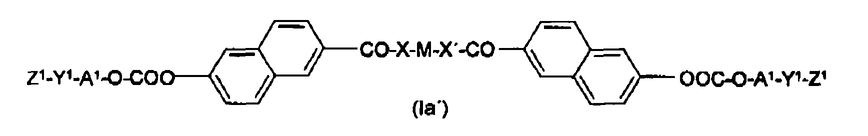 oder der Formel Ib' 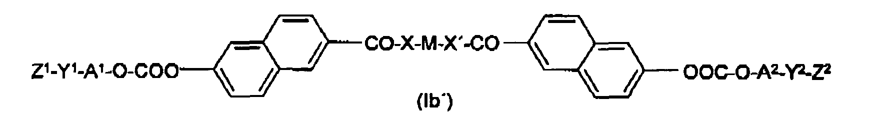 worin M einer Gruppierung der Formel
T²-(Y³- T²-)_{s"}
entspricht, s" Werte von 0 oder 1 annimmt, wobei für s" > 0 die Variablen T² untereinander gleich oder voneinander verschieden sein können, X und X' unabhängig voneinander Sauerstoff oder Schwefel bedeuten und T² und Y³ sowie die übrigen Variablen die Bedeutung wie in den vorhergehenden Ansprüchen definiert besitzen, **dadurch gekennzeichnet, dass** man eine Verbindung der Formel II'
HX-M-X'H II'
mit der entsprechenden Molzahl eines Carbonsäurederivats der Formel IIIa' 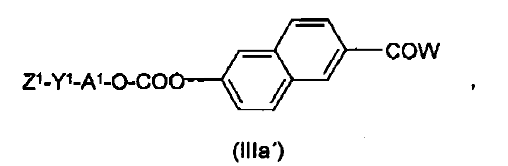 gegebenenfalls in Gegenwart von einer oder mehreren Hilfsverbindungen, zur symmetrischen Verbindung der Formel Ia' umsetzt,
oder dass man eine Verbindung der Formel II' in einem ersten Schritt mit der entsprechenden Molzahl eines Carbonsäurederivats der Formel IIIa', gegebenenfalls in Gegenwart von einer oder mehreren Hilfsverbindungen, und in einem zweiten Schritt mit der entsprechenden Molzahl eines Carbonsäurederivats der Formel IIIb' 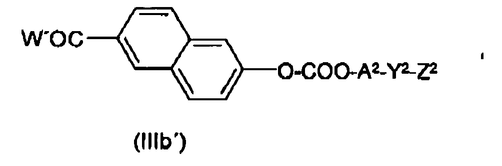 gegebenenfalls in Gegenwart von einer oder mehreren Hilfsverbindungen, zur unsymmetrischen Verbindung der Formel Ib' umsetzt, wobei W und W' für gleiche oder voneinander verschiedene, bei der Umsetzung austretende Abgangsgruppen oder für gleiche oder voneinander verschiedene Vorläufergruppen stehen, welche mit der oder den gegebenenfalls vorliegenden Hilfsverbindungen in eine austretende Abgangsgruppe überführt werden können.

13. Verbindungen der Formel 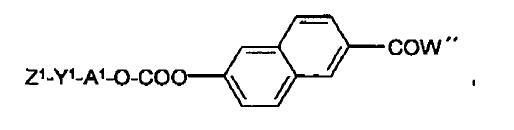 worin W" für Hydroxy, Halogen, C₁-C₄-Alkoxy, teilweise oder vollständig halogeniertes Phenoxy, einen Rest einer anorganischen Säure, einen Rest einer Carbonsäure, einen Rest einer aliphatischen oder aromatischen Sulfonsäure, einen Rest einer teilweise oder vollständig fluorierten aliphatischen oder aromatischen Sulfonsäure oder eine Gruppe der Formel 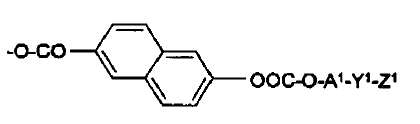 steht, wobei Z¹, Y¹ und A¹ dieselbe Bedeutung wie in den vorhergehenden Ansprüchen definiert besitzen.

14. Verwendung einer erfindungsgemäßen flüssigkristallinen Zusammensetzung gemäß Anspruch 8 oder von Oligomeren oder Polymeren gemäß Anspruch 9 zur Herstellung einer Wärmeisolationsbeschichtung, umfassend eine oder mehrere cholesterische Schichten, die im infraroten Wellenlängenbereich, vorzugsweise oberhalb 750 nm, insbesondere im Wellenlängenbereich von 751 nm bis etwa 2000 nm mindestens 40 %, insbesondere mindestens 45 % der auftreffenden Strahlung reflektiert.

15. Verwendung einer erfindungsgemäßen flüssigkristallinen Zusammensetzung gemäß Anspruch 8 oder von Oligomeren oder Polymeren gemäß Anspruch 9 zur Herstellung einer Wärmeisolationsbeschichtung gemäß Anspruch 14, die im Wellenlängenbereich von etwa 390 nm bis 750 nm eine Transmission von mindestens 80%, insbesondere mindestens 90 % der auftreffenden Strahlung aufweist.

16. Wärmeisolationsbeschichtung, umfassend eine oder mehrere cholesterische Schichten, die im infraroten Wellenlängenbereich, vorzugsweise oberhalb 750 nm, insbesondere im Wellenlängenbereich von 751 nm bis etwa 2000 nm mindestens 40%, insbesondere mindestens 45 % der auftreffenden Strahlung reflektiert, welche unter Verwendung der flüssigkristallinen Zusammensetzung gemäß Anspruch 8 oder der erfindungsgemäßen Oligomeren oder Polymeren gemäß Anspruch 9 erhältlich ist.

17. Wärmeisolationsbeschichtung nach Anspruch 16, die im Wellenlängenbereich von etwa 390 nm bis 750 nm eine Transmission von mindestens 80 %, insbesondere mindestens 90 % der auftreffenden Strahlung aufweist, welche unter Verwendung der flüssigkristallinen Zusammensetzung gemäß Anspruch 8 oder der erfindungsgemäßen Oligomeren oder Polymeren gemäß Anspruch 9 erhältlich ist.

## Claims

1. A compound of the general formula I 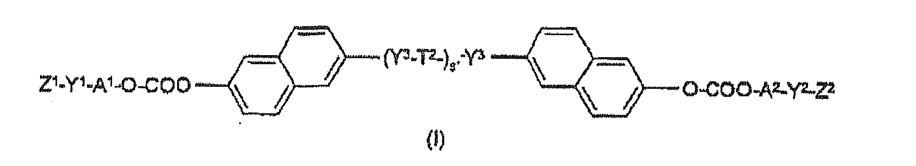 in which the variables are each defined as follows:
Z¹, Z² are each independently hydrogen, optionally substituted C₁-C₂₀-alkyl in which the carbon chain may be interrupted by oxygen atoms in ether function, sulfur atoms in thioether function or by nonadjacent imino or C₁-C₄- alkylimino groups, or reactive radicals by means of which polymerization can be brought about,
A¹ A² are each independently spacers having from 1 to 30 carbon atoms, in which the carbon chain may be interrupted by oxygen atoms in ether function, sulfur atoms in thioether function or by nonadjacent imino or C₁-C₄- alkylimino groups,
Y¹, Y² are each independently a chemical single bond, oxygen, sulfur, -CO-, -O-CO-, -CO-O-, -S-CO-, -CO-S-, -NR-CO- or -CO-NR-,
Y³ is a chemical single bond, oxygen, sulfur, -CR=CR-, -C≡C-, -CR=CR-CO-O-, -O-CO-CR=CR-, -C≡C-O-, -O-C≡-, -CH₂-CH₂-, -CH₂-O-, -O-CH₂-, -CH₂-S-, -S-CH₂-, -CO-, -O-CO-, -CO-O-, -S- CO-, -CO-S-, -NR-CO-, -CO-NR-, -O-CO-O-, - O-CO-NR- or -NR-CO-O-,
R is hydrogen or C₁-C₄-alkyl,
T² is divalent saturated or unsaturated, optionally substituted iso- or heterocyclic radicals,
and
S' is 0, 1 or 2,
where the variables Y³ when s' > 0 and the variables T² when s' > 1 may be the same as one another or different from one another
and
both the hydrogen atoms in the 2,6-naphthyl radical and the hydrogen atoms bonded to carbon atoms in the variables Z¹, Z², A¹, A², Y³, R and T² may be substituted partly or fully by halogen atoms.

2. The compound according to claim 1, in which the variable T² is 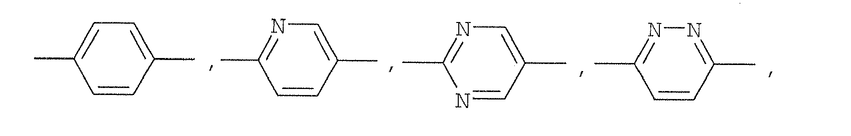 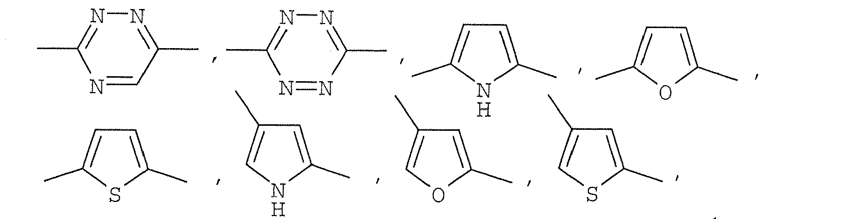 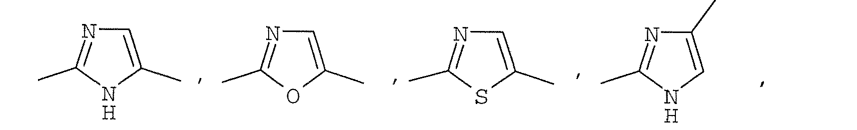 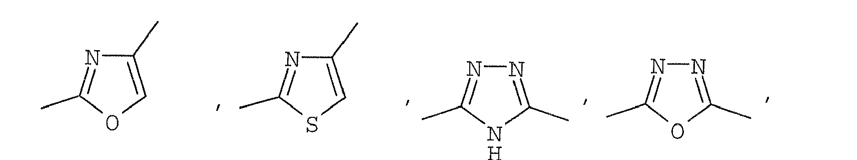 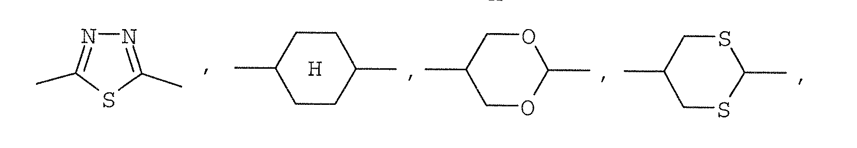 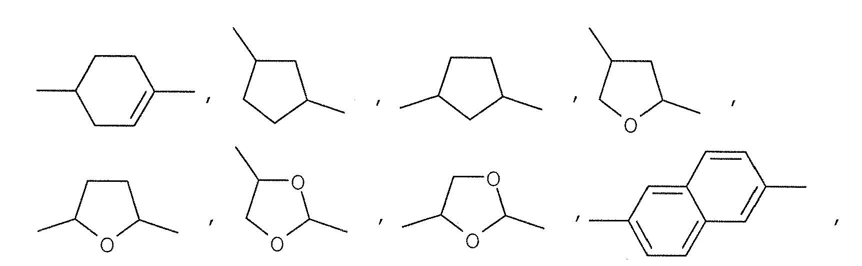 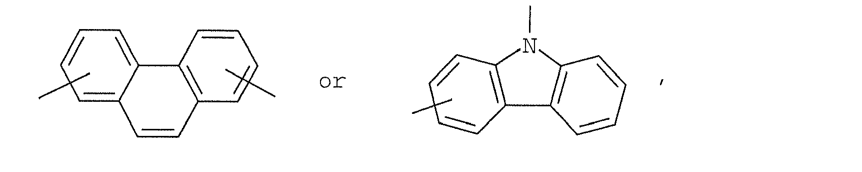 where the radicals 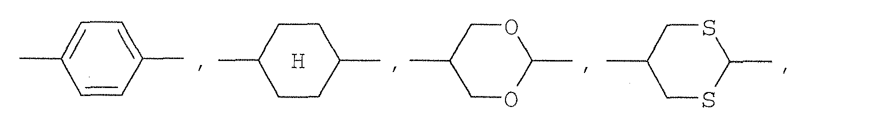 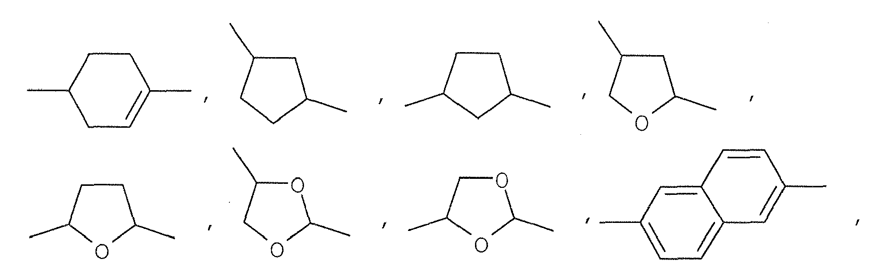 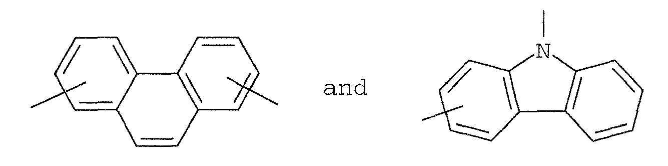 may be substituted by up to four identical or different substituents,
the radical 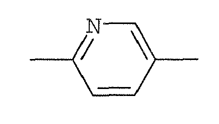 may be substituted by up to three identical or different substituents,
the radicals 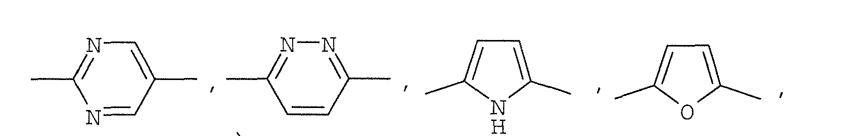 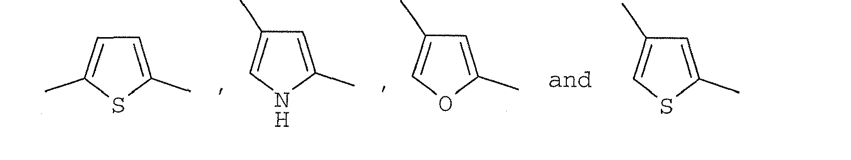 may be substituted by up to two identical or different substituents
and
the radicals 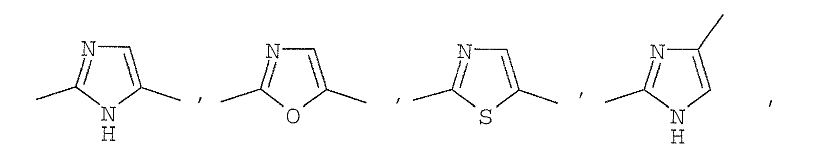 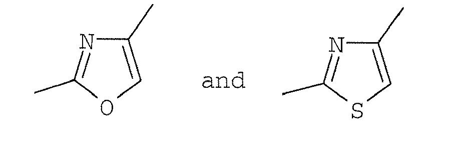 may be substituted by one substituent
halogen, NO₂, NO, CN, CHO, L¹, CO-L¹, X¹-CO-L¹, X¹-SO-L¹, X¹-SO₂-L¹, X¹-L^{1'} CO-X¹-L^{1'}, O-CO-X¹-L^{1'} SOX¹-L^{1'} 1, or SO₂-X¹-L^{1'}, where
L¹ is C₁-C₂₀-alkyl, C₂-C₂₀-alkenyl, C₂-C₂₀- alkynyl , C₆-C₁₀-aryl, heteroaryl having from 2 to 12 carbon atoms, C₆-C₁₀-aryl-C₁-C₂₀- alkyl, C₆-C₁₀-aryl-C₂₋C₂₀-alkenyl, C₆-C₁₀- aryl-C₂-C₂₀-alkynyl, heteroaryl-C₁-C₂₀-alkyl, heteroaryl-C₁-C₂₀-alkenyl or heteroaryl-C₁- C₂₀-alkynyl having in each case from 2 to 12 carbon atoms in the heteroaryl radical, where the C₁-C₂₀ carbon chain may be interrupted by oxygen atoms in ether function, sulfur atoms in thioether function, nonadjacent imino, C₁-C₂₀- alkylimino and/or carbonyl groups, and both the C₆-C₁₀-aryl and the heteroaryl may be substituted by one or more substituents selected from the group consisting of halogen, NO₂, NO, CN, CHO, L², CO-L², X²-CO-L², X²-SO-L², X²-SO₂-L², X²-L^{2'}, CO-X²-L^{2'}, O- CO-X²-L^{2'}, SO-X²-L^{2'} and SO₂-X²-L^{2'},
L^{1'} is hydrogen or, independently of L¹, as defined for L¹,
L² is C₁-C₂₀-alkyl, C₂-C₂₀-alkenyl, C₂-C₂₀- alkynyl, C₆-C₁₀-aryl, heteroaryl having from 2 to 12 carbon atoms, C₆-C₁₀-aryl-C₁-C₂₀- alkyl, C₆-C₁₀-aryl-C₂-C₂₀-alkenyl, C₆-C₁₀- aryl-C₂-C₂₀-alkynyl, heteroaryl-C₁-C₂₀-alkyl, heteroaryl-C₂-C₂₀-alkenyl or heteroaryl-C₂- C₂₀-alkynyl having in each case from 2 to 12 carbon atoms in the heteroaryl radical,
L^{2'} is hydrogen or, independently of L², as defined for L²
and
X¹, X² are each independently oxygen, sulfur or NL^{1'} or NL^{2'},
where some or all of the hydrogen atoms bonded to carbon atoms in the L¹ and/or L² radicals may be substituted by halogen atoms.

3. The compound according to claim 1 or 2, in which the Z¹-Y¹-A¹- and -A²-Y²-Z² moieties are the same.

4. The compound according to one or more of claims 1 to 3, in which Z¹ and/or Z² are reactive radicals

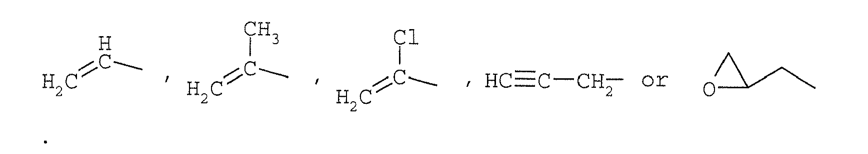

5. The compound according to one or more of claims 1 to 3, in which Z¹ and/or Z² are reactive radicals

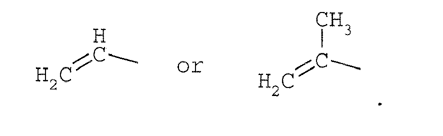

6. The compound according to one or more of claims 1 to 3, in which Z¹-Y¹ and/or Z²-Y² are reactive moieties

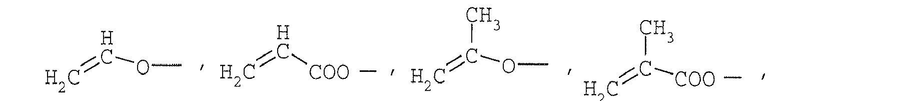

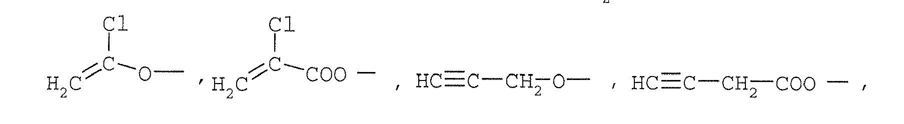

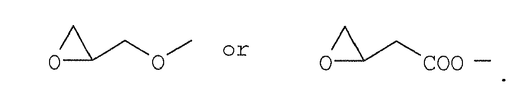

7. The compound according to one or more of claims 1 to 3, in which Z¹-Y¹ and/or Z²-Y² are reactive moieties

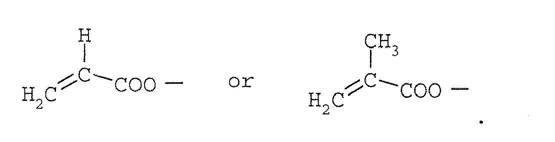

8. A polymerizable or nonpolymerizable liquid-crystalline composition comprising, as components, from 10 to 100% by weight of the compounds according to claims 1 to 7, from 0 to 90% by weight of further monomers, from 0 to 50% by weight of one or more chiral compounds and from 0 to 90% by weight of further additives, the sum of the proportions of the components adding up to 100% by weight.

9. An oligomer or polymer obtainable by oligomerization or polymerization of a polymerizable liquid-crystalline composition according to claim 8.

10. A process for printing or coating a substrate, which comprises applying a polymerizable liquid-crystalline composition according to claim 8 to the substrate and subsequently polymerizing it.

11. The use of a liquid-crystalline composition according to claim 8 or of an oligomer or polymer according to claim 9 for producing optical or electrooptical components.

12. A process for preparing compounds of the formula Ia' 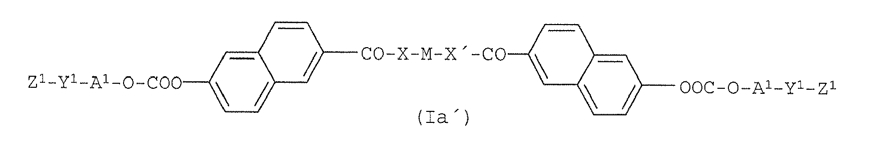 or of the formula Ib' 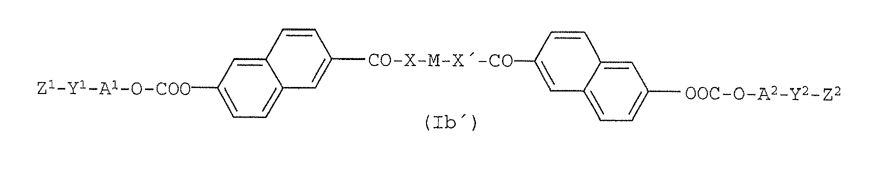 in which M is a moiety of the formula
T²-(Y³-T²-)_{s"},
s" assumes values of 0 or 1, where the variables T² when s " > 0 may be the same as one another or different from one another, X and X' are each independently oxygen or sulfur, and T² and Y³ and also the remaining variables are each as defined in the preceding claims, which comprises reacting a compound of the formula II'
HX-M-X'H II'
with the appropriate number of moles of a carboxylic acid derivative of the formula IIIa' 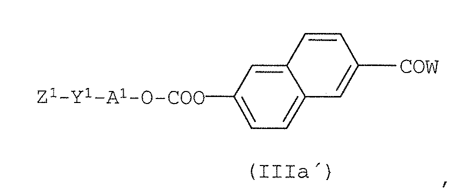 if appropriate in the presence of one or more auxiliary compounds, to give the symmetrical compound of the formula Ia',
or reacting a compound of the formula II', in a first step, with the appropriate number of moles of a carboxylic acid derivative of the formula IIIa', if appropriate in the presence of one or more auxiliary compounds, and, in a second step, with the appropriate number of moles of a carboxylic acid derivative of the formula IIIb' 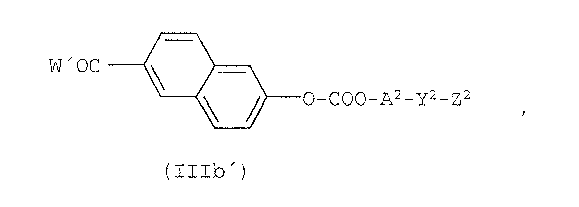 if appropriate in the presence of one or more auxiliary compounds, to give the unsymmetrical compound of the formula Ib', where W and W' are identical or different leaving groups which leave in the reaction or are identical or different precursor groups which can be converted to a leaving group with the auxiliary compound(s) which is/are present if appropriate.

13. A compound of the formula 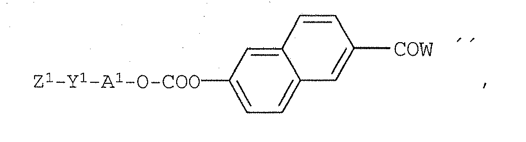 in which W" is hydroxyl, halogen, C₁-C₄-alkoxy, partly or fully halogenated phenoxy, a radical of an inorganic acid, a radical of a carboxylic acid, a radical of an aliphatic or aromatic sulfonic acid, a radical of a partly or fully fluorinated aliphatic or aromatic sulfonic acid or a group of the formula 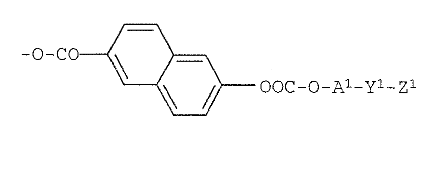 where Z¹, Y¹ and A¹ are each as defined in the preceding claims.

14. The use of an inventive liquid-crystalline composition according to claim 8 or of oligomers or polymers according to claim 9 for producing a thermal insulation coating, comprising one or more cholesteric layers which, in the infrared wavelength region, preferably above 750 nm, in particular in the wavelength range from 751 nm to about 2000 nm, reflects at least 40%, in particular at least 45% of the incident radiation.

15. The use of an inventive liquid-crystalline composition according to claim 8 or of oligomers or polymers according to claim 9 for producing a thermal insulation coating according to claim 14 which, in the wavelength range from about 390 nm to 750 nm, has a transmission of at least 80%, in particular at least 90%, of the incident radiation.

16. A thermal insulation coating comprising one or more cholesteric layers which, in the infrared wavelength range, preferably above 750 nm, in particular in the wavelength range from 751 nm to about 2000 nm, reflects at least 40%, in particular at least 45% of the incident radiation, and which is obtainable using the liquid-crystalline composition according to claim 8 or the inventive oligomers or polymers according to claim 9.

17. The thermal insulation coating according to claim 16, which, in the wavelength range from about 390 nm to 750 nm, has a transmission of at least 80%, in particular at least 90% of the incident radiation, and which is obtainable using the liquid-crystalline composition according to claim 8 or the inventive oligomers or polymers according to claim 9.

## Revendications

1. Composés de formule générale I 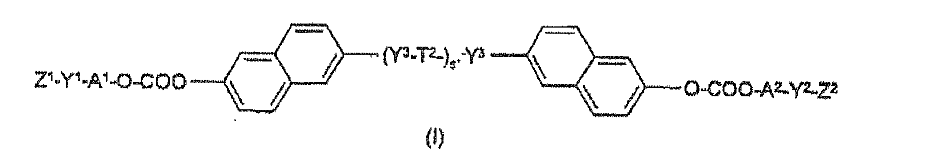 dans laquelle les variables ont les significations suivantes :
Z¹, Z² représentent, indépendamment l'un de l'autre, un atome d'hydrogène, un groupe alkyle en C₁-C₂₀ éventuellement substitué, dans lequel la chaîne carbonée peut être interrompue par des atomes d'oxygène en fonction éther, des atomes de soufre en fonction thioéther ou par des groupes imino ou alkyl(C₁-C₄)imino non contigus, ou des radicaux réactifs, au moyen desquels peut être provoquée une polymérisation,
A¹, A² représentent, indépendamment l'un de l'autre, un espaceur ayant de 1 à 30 atomes de carbone, dans lequel la chaîne carbonée peut être interrompue par des atomes d'oxygène en fonction éther, des atomes de soufre en fonction thioéther ou par des groupes imino ou alkyl(C₁-C₄) imino non contigus,
Y¹, Y² représentent, indépendamment l'un de l'autre, une liaison chimique simple, un atome d'oxygène, un atome de soufre, -CO-, -O-CO-, -CO-O-, -S-CO-, -CO-S-, -NR-CO- ou -CO-NR-,
Y³ représente une liaison chimique simple, un atome d'oxygène, un atome de soufre, -CR=CR-, -C≡C-, -CR=CR-CO-O-, -O-CO-CR=CR-, -C≡C-O-, -O-C≡C-, -CH₂-CH₂-, -CH₂-O-, -O-CH₂-, -CH₂-S-, -S-CH₂-, -CO-, -O-CO-, -CO-O-, -S-CO-, -CO-S-, -NR-CO-, -CO-NR-, -O-CO-O-, -O-CO-NR- ou -NR-CO-O-,
R représente un atome d'hydrogène ou un groupe alkyle en C₁-C₄,
T² représente des radicaux iso- ou hétérocycliques divalents, saturés ou insaturés, éventuellement substitués,
et
s' représente les nombres valant 0, 1 ou 2,
pour s' > 0 les variables Y³ et pour s' > 1 les variables T² pouvant être identiques ou différentes entre elles
et
aussi bien les atomes d'hydrogène dans le radical 2,6-naphtyle que les atomes d'hydrogène liés à des atomes de carbone dans les variables Z¹, Z², A¹, A², Y³, R et T² peuvent être partiellement ou totalement remplacés par des atomes d'halogène.

2. Composés selon la revendication 1, dans lesquels les variables T² représentent 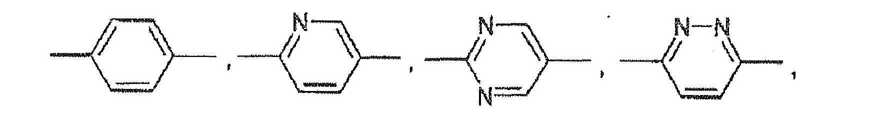 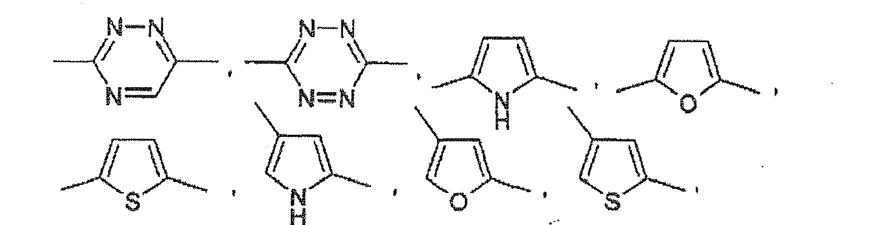 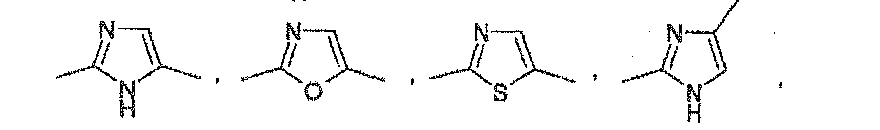 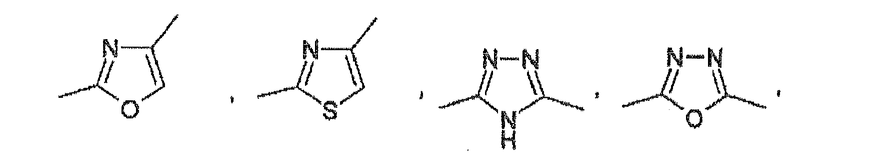 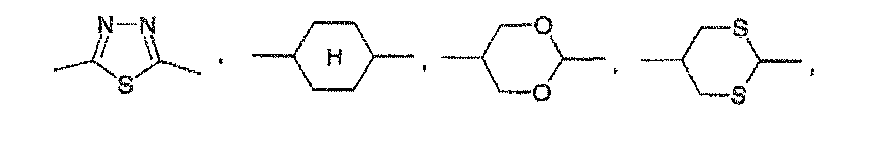 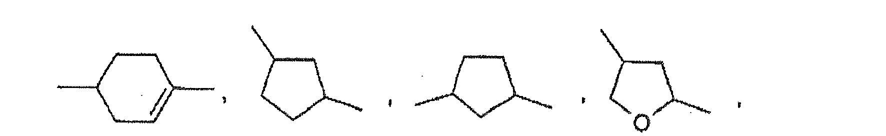 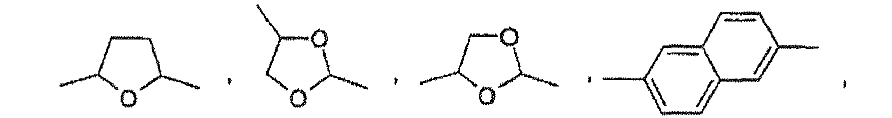 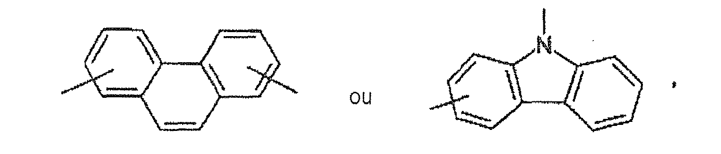 les radicaux 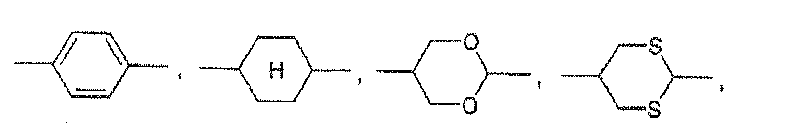 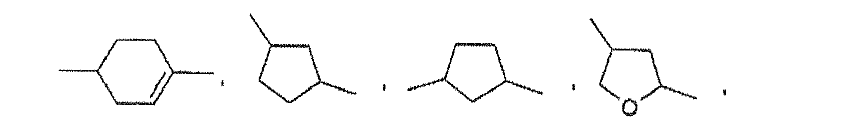 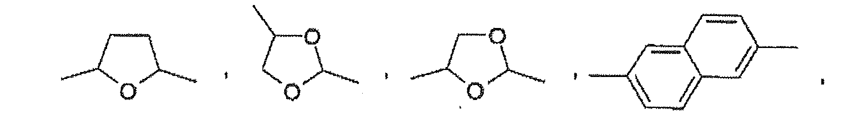 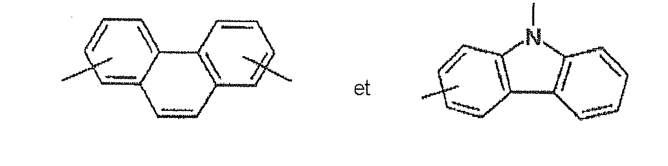 pouvant porter jusqu'à quatre substituants identiques ou différents,
le radical 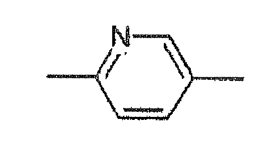 pouvant porter jusqu'à trois substituants identiques ou différents,
les radicaux 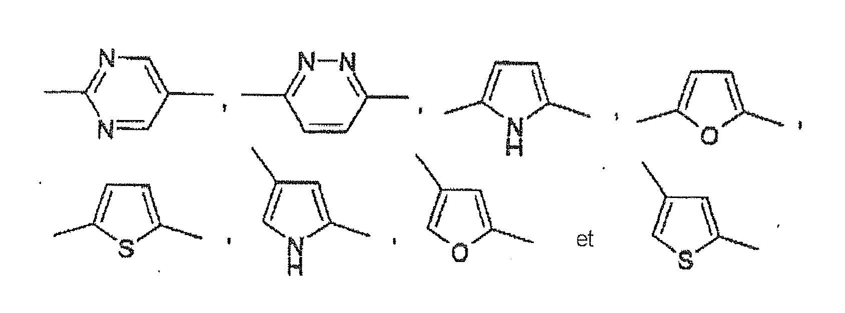 pouvant porter jusqu'à deux substituants identiques ou différents,
et
les radicaux 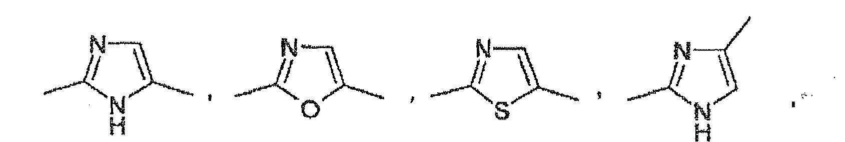 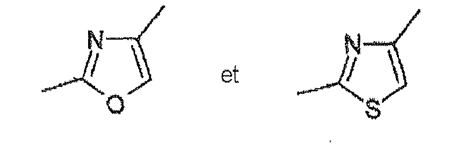 pouvant porter un substituant
halogène, NO₂, NO, CN, CHO, L¹, CO-L¹ , X¹-CO-L¹, X¹-SO-L¹, X¹ -SO₂-L¹, X¹-L^{1'} , CO-X¹-L^{1'} , O-CO-X¹-L^{1'} , SO-X¹-L^{1'} , SO₂-X¹-L^{1'}
L¹ représentant un groupe alkyle en C₁-C₂₀, alcényle en C₂-C₂₀, alcynyle en C₂-C₂₀, aryle en C₆-C₁₀, hétéroaryle ayant de 2 à 12 atomes de carbone, aryl(C₆-C₁₀)-alkyle(C₁-C₂₀), aryl(C₆-C₁₀)-alcényle(C₂-C₂₀), aryl(C₆-C₁₀)- alcynyle(C₂-C₂₀), hétéroaryl-alkyle(C₁-C₂₀), hétéroaryl-alcényle(C₁-C₂₀) ou hétéroaryl- alcynyle(C₁-C₂₀) ayant chacun de 2 à 12 atomes de carbone dans le radical hétéroaryle, la chaîne carbonée en C₁-C₂₀ pouvant être interrompue par des atomes d'oxygène en fonction éther, des atomes de soufre en fonction thioéther, des groupes imino, alkyl(C₁-C₂₀)imino et/ou carbonyle non contigus et aussi bien le radical aryle en C₆-C₁₀ que le radical hétéroaryle pouvant porter un ou plusieurs substituants choisis dans l'ensemble constitué par des atomes d'halogène et des groupes NO₂, NO, CN, CHO, L², CO-L², X²-CO-L², X²-SO-L², X²-SO₂-L², X²-L^{2'}, CO-X²-L^{2'}, O-CC-X²-L^{2'}, SO-X²-L^{2'} et SO₂-X²-L^{2'} ,
L^{1'} représentant un atome d'hydrogène ou ayant, indépendamment de L¹, la même signification que L¹,
L² représentant un groupe alkyle en C₁-C₂₀, alcényle en C₂-C₂₀, alcynyle en C₂-C₂₀, aryle en C₆-C₁₀, hétéroaryle ayant de 2 à 12 atomes de carbone, aryl(C₆-C₁₀)-alkyle(C₁-C₂₀), aryl(C₆-C₁₀)-alcényle(C₂-C₁₀), aryl(C₆-C₁₀)- alcynyle(C₂-C₂₀), hétéroaryl-alkyle(C₁-C₂₀), hétéroaryl-alcényle(C₂C₂₀) ou hétéroaryl- alcynyle(C₂-C₂₀) ayant chacun de 2 à 12 atomes de carbone dans le radical hétéroaryle,
L^{2'} représentant un atome d'hydrogène ou ayant, indépendamment de L² la même signification que L²
et
X¹, X² représentant, indépendamment l'un de l'autre, un atome d'oxygène, un atome de soufre ou NL^{1'} ou NL^{2'},
dans les radicaux L¹ et/ou L² les atomes d'hydrogène liés à des atomes de carbone pouvant être partiellement ou totalement remplacés par des atomes d'halogène.

3. Composés selon la revendication 1 ou 2, dans lesquels les groupements Z¹-Y¹-A¹- et -A²-Y²-Z² sont identiques.

4. Composés selon une ou plusieurs des revendications 1 à 3, dans lesquels Z¹ et/ou Z² représente(nt) les radicaux réactifs

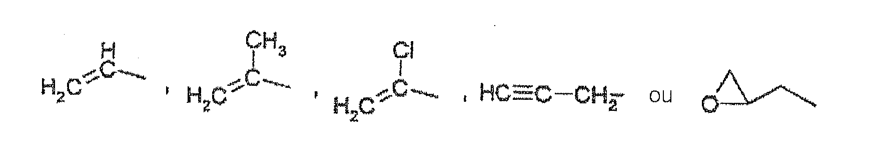

5. Composés selon une ou plusieurs des revendications 1 à 3, dans lesquels Z¹ et/ou Z² représente(nt) les radicaux réactifs

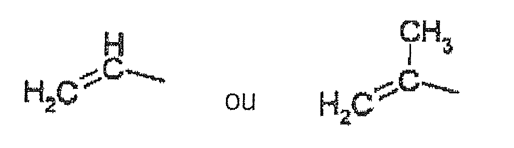

6. Composés selon une ou plusieurs des revendications 1 à 3, dans lesquels Z¹-Y¹ et/ou Z²-Y² représente(nt) les radicaux réactifs 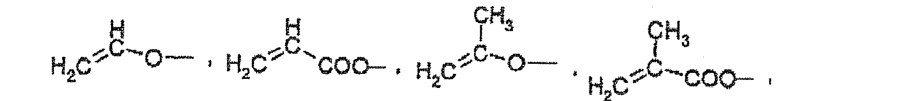 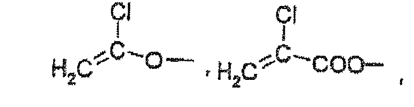 HC≡C-CH₂-O- HC≡C-CH₂-COO-,

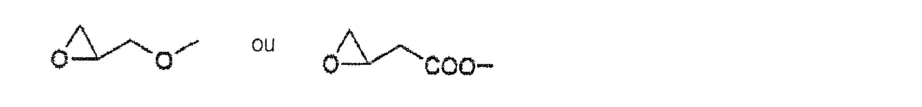

7. Composés selon une ou plusieurs des revendications 1 à 3, dans lesquels Z¹-Y¹ et/ou Z²-Y² représente(nt) les groupements réactifs

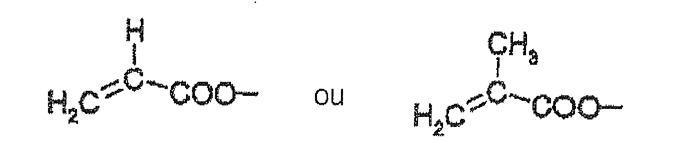

8. Composition de type cristal liquide polymérisable ou non polymérisable, contenant en tant que composants 10 à 100 % en poids des composés selon les revendications 1 à 7, 0 à 90 % en poids d'autres monomères, 0 à 50 % en poids d'un ou plusieurs composés chiraux et 0 à 90 % en poids d'autres additions, la somme des proportions des composants étant égale à 100 % en poids.

9. Oligomère ou polymère, pouvant être obtenu par oligomérisation ou polymérisation d'une composition de type cristal liquide polymérisable, selon la revendication 8.

10. Procédé pour l'impression ou le revêtement d'un support, **caractérisé en ce qu'**on applique sur le support une composition de type cristal liquide polymérisable, selon la revendication 8, et ensuite on la polymérise.

11. Utilisation d'une composition de type cristal liquide selon la revendication 8 ou d'un oligomère ou polymère selon la revendication 9, pour la production de composants optiques ou électro-optiques.

12. Procédé pour la préparation de composés de formule Ia' 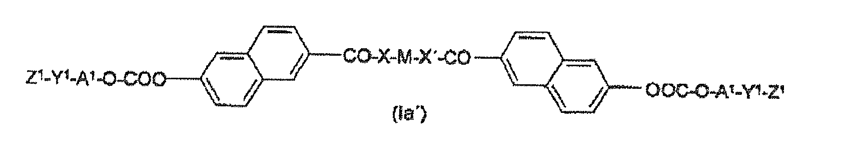 ou de formule Ib' 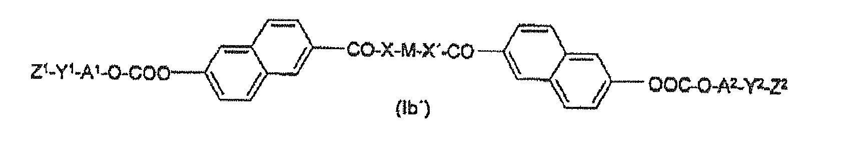 où M correspond à un groupement de formule
T²- (Y³-T²-)_{s"}
s'' a la valeur 0 ou 1, pour s'' > 0 les variables T² pouvant être identiques ou différentes entre elles, X et X' représentent, indépendamment l'un de l'autre, un atome d'oxygène ou de soufre et T² et Y³ ainsi que les autres variables ayant la signification telle que définie dans les revendications précédentes, **caractérisé en ce qu'**on fait réagir un composé de formule II'
HX-M-X'H II'
avec le nombre de moles correspondant d'un dérivé d'acide carboxylique de formule IIIa' 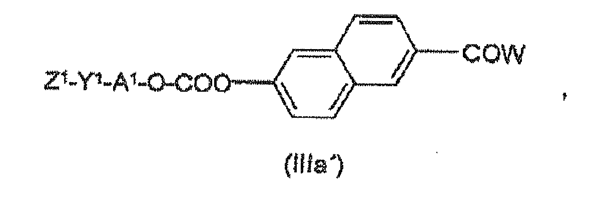 éventuellement en présence d'un ou plusieurs composés auxiliaires, pour l'obtention du composé symétrique de formule Ia',
ou **en ce qu'**on fait réagir dans une première étape un composé de formule II' avec le nombre de moles correspondant d'un dérivé d'acide carboxylique de formule IIIa', éventuellement en présence d'un ou plusieurs composés auxiliaires, et dans une deuxième étape avec le nombre de moles correspondant d'un dérivé d'acide carboxylique de formule IIIb' 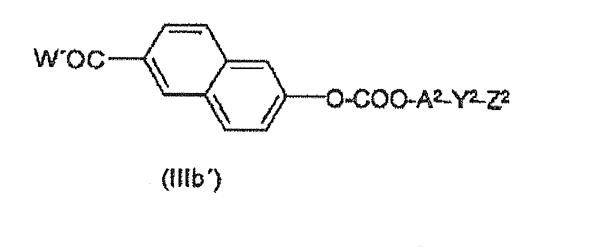 éventuellement en présence d'un ou plusieurs composés auxiliaires, pour l'obtention du composé asymétrique de formule Ib', W et W' représentant des groupes séparables identiques ou différents l'un de l'autre, partant lors de la réaction, ou des groupes précurseurs identiques ou différents l'un de l'autre, qui, avec le ou les composés auxiliaires éventuellement présents, peuvent être convertis en un groupe séparable partant.

13. Composés de formule 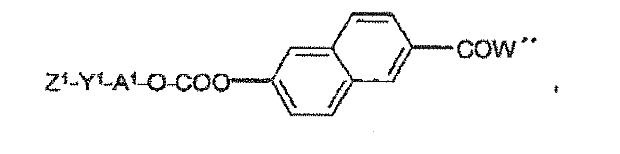 dans laquelle W" représente un groupe hydroxy, un atome d'halogène, un groupe alcoxy en C₁-C₄, un groupe phénoxy partiellement ou totalement halogéné, un reste d'un acide inorganique, un reste d'un acide carboxylique, un reste d'un acide sulfonique aliphatique ou aromatique, un reste d'acide sulfonique aliphatique ou aromatique partiellement ou totalement fluoré ou un groupe de formule 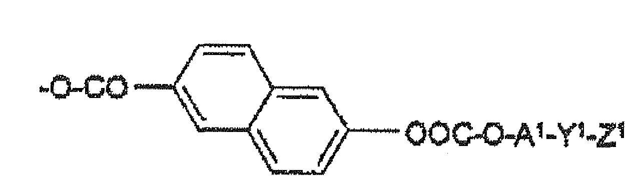 dans laquelle Z¹, Y¹ et A¹ ont les mêmes significations que dans les revendications précédentes.

14. Utilisation d'une composition de type cristal liquide conforme à l'invention, selon la revendication 8, ou d'oligomères ou de polymères selon la revendication 9, pour la production d'un revêtement isolant thermique, comprenant une ou plusieurs couches cholestériques qui dans la plage de longueurs d'onde infrarouges, de préférence au-dessus de 750 nm, en particulier dans la plage de longueurs d'onde de 751 nm à environ 2 000 nm, réfléchissent au moins 40 %, en particulier au moins 45 % du rayonnement incident.

15. Utilisation d'une composition de type cristal liquide conforme à l'invention, selon la revendication 8, ou d'oligomères ou de polymères selon la revendication 9, pour la production d'un revêtement isolant thermique selon la revendication 14, qui dans la plage de longueurs d'onde d'environ 390 nm à 750 nm présente une transmission d'au moins 80 %, en particulier d'au moins 90 % du rayonnement incident.

16. Revêtement isolant thermique, comprenant une ou plusieurs couches cholestériques qui dans la plage de longueurs d'onde infrarouges, de préférence au-dessus de 750 nm, en particulier dans la plage de longueurs d'onde de 751 nm à environ 2 000 nm, réfléchissent au moins 40 %, en particulier au moins 45 % du rayonnement incident, qui peut être obtenu avec utilisation de la composition de type cristal liquide selon la revendication 8 ou des oligomères ou polymères conformes à l'invention selon la revendication 9.

17. Revêtement isolant thermique selon la revendication 16, qui dans la plage de longueurs d'onde d'environ 390 à 750 nm présente une transmission d'au moins 80 %, en particulier d'au moins 90 % du rayonnement incident, qui peut être obtenu avec utilisation de la composition de type cristal liquide selon la revendication 8 ou des oligomères ou polymères conformes à l'invention selon la revendication 9.
